# EUROPEAN PATENT APPLICATION

(11) **EP 4 640 841 A1**
(43) Date of publication of application: **29.10.2025**
(21) Application number: 24172148.9
(22) Date of filing: 24.04.2024
(51) Int. Cl.: C12Q 1/66, G01N 33/537, G01N 33/569, G01N 33/68

(54) **IMMUNO THERMAL SHIFT ASSAY**

(71) Applicant: Universität Münster, 48149 Münster (DE)
(72) Inventor: KUEHN, Joachim, 48151 Muenster (DE); CLASSEN, Nica, 48149 Muenster (DE)
(74) Representative: Schiweck Weinzierl Koch Patentanwälte Partnerschaft mbB

(57) **Abstract**

The present invention is directed to a method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii), wherein the ratio of the detected signal (i) and (ii) allows determining the binding characteristic between said antibody and said target polypeptide, and thereby the immune status of the subject is determined. Further, the present invention is directed to the use of the method of the invention for determining one or more of the following antibody maturation, vaccination status, antibody determination in acute, chronic or anamnestic infection, antibody effectiveness, antibody affinity and/or antibody avidity. Finally, the present invention is directed to a kit comprising a mixture comprising a fusion protein of a target peptide and a labeling peptide for conducting the method of the present invention.

## Description

### TECHNICAL FIELD OF THE INVENTION

The present invention relates to a method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii), wherein the ratio of the detected signal (i) and (ii) allows determining the binding characteristic between said antibody and said target polypeptide, and thereby the immune status of the subject is determined. The present invention relates also to the use of the method for determining one or more of the following antibody maturation, immune status, vaccination status, antibody determination in acute, chronic or anamnestic infection, antibody effectiveness, antibody affinity, and/or antibody avidity. Further, the present invention relates to a kit comprising a mixture comprising a fusion protein of a target peptide and a labeling peptide for use in determining the binding property of an antibody for the target peptide.

### BACKGROUND OF THE INVENTION

Thermal shift assays (TSA), synonymous for differential scanning fluorimetry (DSF), have extensively been used throughout the past years and evolved as important tool in drug discovery and biochemical interaction studies, especially to qualify interactions of small molecules with a target protein or between two proteins [1] (Brandts, J.F.; Lin, L.N. Study of Strong to Ultratight Protein Interactions Using Differential Scanning Calorimetry. Biochemistry 1990, 29, 6927-6940, doi:10.1021/bi00481a024.). The technique depends on the thermodynamical stabilization of a protein by binding to a ligand. This conformational stabilization is caused by van-der-Waals forces, hydrogen bonds, dipole-dipole and electrostatic interactions, inducing a negative change of G (Gibbs free energy) of the complex' initial state and increasing the activation energy needed to provoke protein denaturation or unfolding [2,3] (Bischof, J.C.; He, X. Thermal Stability of Proteins. 2005, 33, 12-33, doi:10.1196/annals.1363.003; Du, X.; Li, Y.; Xia, Y.L.; Ai, S.M.; Liang, J.; Sang, P.; Ji, X.L.; Liu, S.Q. Insights into protein-ligand interactions: Mechanisms, models, and methods. Int. J. Mol. Sci. 2016, 17, doi:10.3390/ijms17020144.). Readout of protein stability can be conducted by adding fluorescent dyes like SYPRO orange or ANS interacting with hydrophobic regions of the protein (reviewed by [4] (Hawe, A.; Sutter, M.; Jiskoot, W. Extrinsic fluorescent dyes as tools for protein characterization. Pharm. Res. 2008, 25, 1487-1499, doi: 10.1007/s11095-007-9516-9.)), or by measuring the intrinsic fluorescence activity of tryptophan residues [5] (Eftink, M.R. The use of fluorescence methods to monitor unfolding transitions in proteins. Biophys. J. 1994, 66, 482-501, doi:10.1016/s0006-3495(94)80799-4.). The first thermal shift assay designed for drug discovery was published in 2001 [6] (Pantoliano, M.W.; Petrella, E.C.; Kwasnoski, J.D.; Lobanov, V.S.; Myslik, J.; Graf, E.; Carver, T.; Asel, E.; Springer, B.A.; Lane, P.; et al. High-Density Miniaturized Thermal Shift Assays as a General Strategy for Drug Discovery. J. Biomol. Screen. 2001, 6, 429-440, doi:10,1177/108705710100600609.), using a fluorescent protein-binding dye enabling rapid screening and easy readout of small molecule ligands. Many improvements have been made optimizing the dye-based methods for drug discovery and elucidating the mechanisms of action and interpretation of results, as well for protein-small molecule interactions as for protein-protein interactions ([7-11]) (Lo, M.C.; Aulabaugh, A.; Jin, G.; Cowling, R.; Bard, J.; Malamas, M.; Ellestad, G. Evaluation of fluorescence-based thermal shift assays for hit identification in drug discovery. Anal. Biochem. 2004, 332, 153-159, doi:10.1016/j.ab.2004.04.031; Niesen, F.H.; Berglund, H.; Vedadi, M. The use of differential scanning fluorimetry to detect ligand interactions that promote protein stability. Nat. Protoc. 2007, 2, 2212-2221, doi:10.1038/nprot.2007.321; Layton, C.J.; Hellinga, H.W. Thermodynamic analysis of ligand-induced changes in protein thermal unfolding applied to high-throughput determination of ligand affinities with extrinsic fluorescent dyes. Biochemistry 2010, 49, 10831-10841, doi:10.1021/bi101414z; Layton, C.J.; Hellinga, H.W. Quantitation of protein-protein interactions by thermal stability shift analysis. Protein Sci. 2011, 20, 1439-1450, doi:10.1002/pro.674; Ericsson, U.B.; Hallberg, B.M.; DeTitta, G.T.; Dekker, N.; Nordlund, P. Thermofluor-based high-throughput stability optimization of proteins for structural studies. Anal. Biochem. 2006, 357, 289-298, doi:10.1016/j.ab.2006.07.027.). The value of TSA increased especially for biochemical applications by development of cellular thermal shift assay (CETSA) methods, enabling direct measurements in crude cell lysates, biological tissues or supernatants, however, requiring immunoblot analysis of the target protein or mass spectrometry [12-14] (Jafari, R.; Almqvist, H.; Axelsson, H.; Ignatushchenko, M.; Lundbäck, T.; Nordlund, P.; Molina, D.M. The cellular thermal shift assay for evaluating drug target interactions in cells. Nat. Protoc. 2014, 9, 2100-2122, doi:10.1038/nprot.2014.138; Molina, D.M.; Jafari, R.; Ignatushchenko, M.; Seki, T.; Larsson, E.A.; Dan, C.; Sreekumar, L.; Cao, Y.; Nordlund, P. Monitoring drug target engagement in cells and tissues using the cellular thermal shift assay. Science. 2013, 341, 84-87, doi:10.1126/science.1233606; Savitski, M.M.; Reinhard, F.B.M.; Franken, H.; Werner, T.; Savitski, M.F.; Eberhard, D.; Molina, D.M.; Jafari, R.; Dovega, R.B.; Klaeger, S.; et al. Tracking cancer drugs in living cells by thermal profiling of the proteome. Science. 2014, 346, doi:10.1126/science.1255784.). These assays allow investigation of interactions inside an organism, and can serve as tool for drug discovery, industrial or biological applications and research, including proteome analysis [15-18] (Tolvanen, T.A. Current Advances in CETSA. Front. Mol. Biosci. 2022, 9, doi:10.3389/fmolb.2022.866764; Franken, H.; Mathieson, T.; Childs, D.; Sweetman, G.M.A.; Werner, T.; Tögel, I.; Doce, C.; Gade, S.; Bantscheff, M.; Drewes, G.; et al. Thermal proteome profiling for unbiased identification of direct and indirect drug targets using multiplexed quantitative mass spectrometry. Nat. Protoc. 2015, 10, 1567-1593, doi:10.1038/nprot.2015.101; Jarzab, A.; Kurzawa, N.; Hopf, T.; Moerch, M.; Zecha, J.; Leijten, N.; Bian, Y.; Musiol, E.; Maschberger, M.; Stoehr, G.; et al. Meltome atlas-thermal proteome stability across the tree of life. Nat. Methods 2020, 17, 495-503, doi:10.1038/s41592-020-0801-4; Henderson, M.J.; Holbert, M.A.; Simeonov, A.; Kallal, L.A. High-Throughput Cellular Thermal Shift Assays in Research and Drug Discovery. SLAS Discov. 2020, 25, 137-147, doi:10.1177/2472555219877183.).

Approaches modifying the readout system have been made, e.g. by staining with fluorescent secondary antibody [19] (Massey, A.J. A high content, high throughput cellular thermal stability assay for measuring drug-target engagement in living cells. PLoS One 2018, 13, 1-17, doi:10.1371/journal.pone.0195050.) or using receptor and donor beads causing a fluorescence signal when binding the target, called AlphaScreen [20,21] (Almqvist, H.; Axelsson, H.; Jafari, R.; Dan, C.; Mateus, A.; Haraldsson, M.; Larsson, A.; Molina, D.M.; Artursson, P.; Lundbäck, T.; et al. CETSA screening identifies known and novel thymidylate synthase inhibitors and slow intracellular activation of 5-fluorouracil. Nat. Commun. 2016, 7, 1-11, doi:10.1038/ncomms11040; Shaw, J.; Dale, I.; Hemsley, P.; Leach, L.; Dekki, N.; Orme, J.P.; Talbot, V.; Narvaez, A.J.; Bista, M.; Martinez Molina, D.; et al. Positioning High-Throughput CETSA in Early Drug Discovery through Screening against B-Raf and PARP1. SLAS Discov. 2019, 24, 121-132, doi:10.1177/2472555218813332.).

Further methods have been developed using thermal stable reporter proteins genetically fused to the target protein. By this approach, the folding status of the target protein is linked to the reporter activity and enables quantitation of the percentage of intact target protein in solution. A particularly suitable reporter protein is nanoluciferase (NanoLuc), a high activity, temperature-stable small luciferase [22-24] (Dart, M.L.; Machleidt, T.; Jost, E.; Schwinn, M.K.; Robers, M.B.; Shi, C.; Kirkland, T.A.; Killoran, M.P.; Wilkinson, J.M.; Hartnett, J.R.; et al. Homogeneous Assay for Target Engagement Utilizing Bioluminescent Thermal Shift. ACS Med. Chem. Lett. 2018, 9, 546-551, doi:10.1021/acsmedchemlett.8b00081; Martinez, N.J.; Asawa, R.R.; Cyr, M.G.; Zakharov, A.; Urban, D.J.; Roth, J.S.; Wallgren, E.; Klumpp-Thomas, C.; Coussens, N.P.; Rai, G.; et al. A widely-applicable high-throughput cellular thermal shift assay (CETSA) using split Nano Luciferase. Sci. Rep. 2018, 8, doi:10.1038/s41598-018-27834-y; England, C.G.; Ehlerding, E.B.; Cai, W. NanoLuc: A Small Luciferase Is Brightening Up the Field of Bioluminescence. Bioconjug. Chem. 2016, 27, 1175-1187, doi:10.1021/acs.bioconjchem.6b00112.). Either the whole enzyme can be fused to the target protein, or only one domain, adding the second domain after heating with the substrate (SplitLuc CETSA) [23] (Martinez, N.J.; Asawa, R.R.; Cyr, M.G.; Zakharov, A.; Urban, D.J.; Roth, J.S.; Wallgren, E.; Klumpp-Thomas, C.; Coussens, N.P.; Rai, G.; et al. A widely-applicable high-throughput cellular thermal shift assay (CETSA) using split Nano Luciferase. Sci. Rep. 2018, 8, doi:10.1038/s41598-018-27834-y.). The principle of enzyme fragment complementation (EFC) is also used in a β-galactosidase-based approach [25] (McNulty, D.E.; Bonnette, W.G.; Qi, H.; Wang, L.; Ho, T.F.; Waszkiewicz, A.; Kallal, L.A.; Nagarajan, R.P.; Stern, M.; Quinn, A.M.; et al. A High-Throughput Dose-Response Cellular Thermal Shift Assay for Rapid Screening of Drug Target Engagement in Living Cells, Exemplified Using SMYD3 and IDO1. SLAS Discov. 2018, 23, 34-46, doi:10.1177/2472555217732014.). Both approaches are resulting in luminescence signals simply to quantify. Including a reporter enzyme into TSA improves the workflow by enabling heating and measurement of the target protein without a complicated protein separation or MS analysis. The method has been further optimized by enabling intracellular engagement of target protein and small molecule ligand (BiTSA) [26] (Mortison, J.D.; Cornella-Taracido, I.; Venkatchalam, G.; Partridge, A.W.; Siriwardana, N.; Bushell, S.M. Rapid Evaluation of Small Molecule Cellular Target Engagement with a Luminescent Thermal Shift Assay. ACS Med. Chem. Lett. 2021, 12, 1288-1294, doi:10.1021/acsmedchemlett.1c00276.).

First approaches have been made to also correlate ligand affinity to thermal stability of antigen/antibody complexes [27] (Kirley, T.L.; Norman, A.B.; Wetzel, H.N. A novel differential scanning fluorimetry analysis of a humanized anti-cocaine mAb and its ligand binding characteristics. J. Immunol. Methods 2020, 476, doi:10.1016/j.jim.2019.112676.), but it appears that only purified antibodies were used in TSA. Namely, reporter enzyme-based assays have not been applied for investigation of antibody binding kinetics and strength, and polyclonal serum samples have not been analyzed by these methods.

To detect and specify the humoral response in patients, different serological methods serve as useful tools, enabling classification and quantification of specific antibodies from human samples. Determination of antibodies in blood serum and other body fluids can give information about status of acute or chronic infections, on response to vaccination or on immunologic status related to a specific pathogen. The most common technique to quantify antibody levels *in vitro* are solid-phase immunoassays such as ELISA, which allow not only quantitation but also determination of antigen classes by varying the second antibody. Although ELISA-based assays are used as workhorses for the determination of antibody levels, these tests neither give information about the ability of the antibodies detected to neutralize function of the antigenic structure, nor do they indicate binding strength or affinity maturation of antibodies. Hence, the results obtained in quantitative assays only roughly correlate with the antibody quality and function. Determination of authentic virus-specific neutralizing antibodies is inherently complicated by the need to work with infectious agents. To investigate neutralization ability of antibodies *in vitro,* surrogate virus neutralization tests (sVNTs) represent a useful tool, giving information about antibodies directly inhibiting antigen-target interaction. But also, they do not directly measure antibody avidity, nor do they detect non-neutralizing antibodies, which may also play an important role in immune response. Additionally, the vast majority of routine serological assays are solid phase-based, which affects association and dissociation behavior of antibody/antigen complexes. Antibody affinity describes the binding strength of a single paratope of an antibody bound to the corresponding epitope of the antigen, whilst antibody avidity or functional affinity describes the total binding strength of multivalent antibodies to multivalent antigens, which could increase the total binding energy by several orders of magnitude. Antibody avidity presumably is a useful parameter for determining the time point of infections, to investigate antibody affinity maturation and to evaluate effectiveness of vaccinations. Determination of antibody avidity is a common method for monitoring several infections, e.g., for rubella, Epstein Barr virus, cytomegalovirus or toxoplasmosis [28] (Hedman, K.; Lappalainen, M.; Söderlund, M.; Hedman, L. Avidity of IgG in serodiagnosis of infectious diseases. Rev. Med. Microbiol. 1993, 4, 123-129, doi:10.1097/00013542-199307000-00001.). Measurement of antibody avidity in human samples comprises multiple difficulties because thermodynamic interaction studies of antigen and antibody are commonly carried out using purified target structures and a single monoclonal antibody, which makes these methods not usable for routine diagnostics. Commercially available avidity test kits mainly rely on ELISA-based readout systems [29] (Friguet, B.; Chaffotte, A.F.; Djavadi-ohaniance, L.; Goldberg, M.E. Measurements of the true affinity constant in solution of antigen-antibody complexes by ELISA. J. Immunol. Methods 1985, 77, 305-319, doi:10.1016/0022-1759(85)90044-4.), and mandatorily include an assay step using chaotropic agents like urea [30] (Hedman, K.; Rousseau, S.A. Measurement of avidity of specific IgG for verification of recent primary rubella. J. Med. Virol. 1989, 27, 288-292, doi:10.1002/jmv.1890270406.), guanidinium or diethylamine [31] (Thomas, H.I.J.; Morgan-Capner, P. Rubella-specific IgG subclass avidity ELISA and its role in the differentiation between primary rubella and rubella reinfection. Epidemiol. Infect. 1988, 101, 591-598, doi:10.1017/S0950268800029459.) to disrupt binding and inhibit reactivity of low-avidity antibodies. As result, an avidity index of treated/untreated samples is obtained, giving the quantity of high-avidity antibodies in relation to the total amount of binding antibodies. By using indirect ELISA, the obstacle of using a solid phase can in parts be overcome. Other methods for measuring antibody avidity are readout by radio immunoassays (RIA), measuring dissociation constants by equilibrium dialysis [32] (Eisen, H.N.; Siskind, G.W. Variations in Affinities of Antibodies during the Immune Response. Biochemistry 1964, 3, 996-1008, doi:10.1021/bi00895a027.) or surface plasmon resonance [33] (Lynch, H.E.; Stewart, S.M.; Kepler, T.B.; Sempowski, G.D.; Alam, S.M. Surface plasmon resonance measurements of plasma antibody avidity during primary and secondary responses to anthrax protective antigen. J. Immunol. Methods 2014, 404, 1-12, doi:10.1016/j.jim.2013.11.026.), of which the first one suffers from dependence on radioactive reagents, and the latter are not suited for large ligands, complex matrices and a large number of samples.

In view of these drawbacks of the existing methods, the underlying technical problem is the provision of a method which provides an improved determination of the immune status of a subject, which allows the determination of antibody binding strength and which method is suitable for routine diagnostic.

This technical problem is solved with the enclosed set of claims.

### SUMMARY

The present invention is directed to a method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii), wherein the ratio of the detected signal (i) and (ii) allows determining the binding characteristic between said antibody and said target polypeptide, and thereby the immune status of the subject is determined.

The present invention is also directed to the use of the method of the invention for determining one or more of the following - antibody maturation, immune status, vaccination status, antibody determination in acute, chronic or anamnestic infection, antibody effectiveness, antibody affinity and/or antibody avidity.

Further, the present invention is directed to a kit comprising a mixture comprising a fusion protein of a target peptide and a labeling peptide for use in determining the binding property of a ligand of interest specific for the target peptide.

### BRIEF DESCRIPTION OF THE DRAWINGS

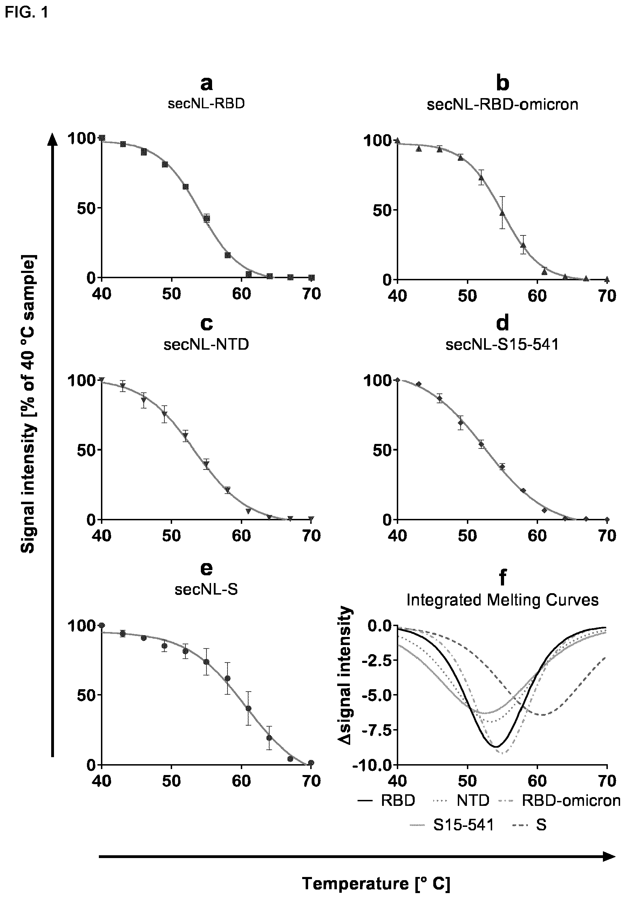

**Figure 1****:** NLuc-S constructs diluted in sample buffer were heated to temperatures from 40-70°C for 3 min in a PCR cycler. Indicated are melting curves of the different NLuc-S fusion protein constructs **(a)** secNL-RBD, (b) secNL-RBD-omicron (BA.1), **(c)** secNL-NTD, **(d)** secNL-S15-541 and **(e)** secNL-S. Calculating the first derivatives of the melting curves resulted in melting peaks **(f).** Values were normalized to 40°C value of each experiment series. Means ± SD from n=3 independent experiments are shown. Curves were calculated in GraphPad Prism software using nonlinear regression/Boltzmann sigmoidal curve fit.

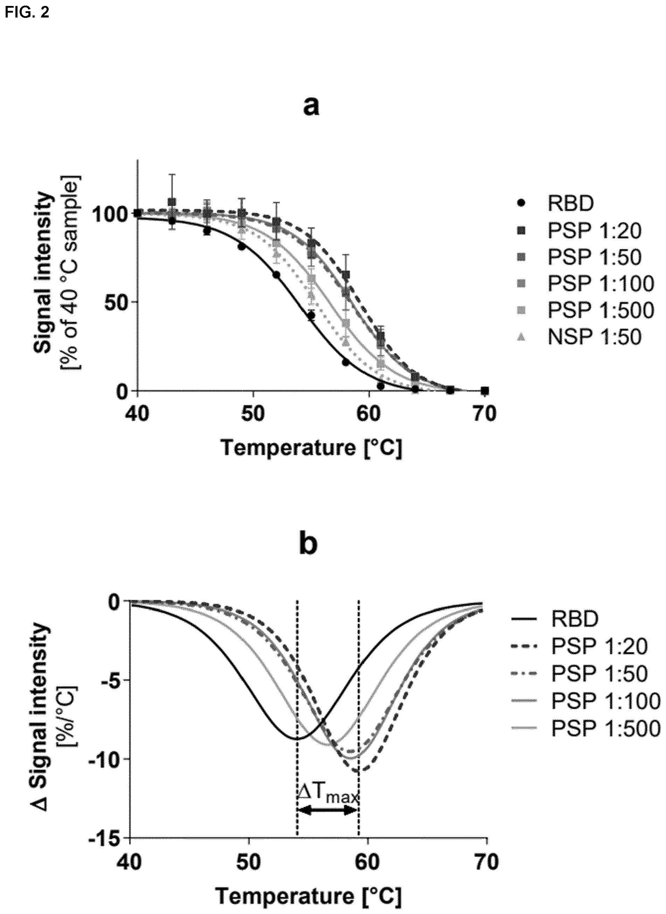

**Figure 2****:** Effect of anti-S positive human serum pool (PSP) on melting behavior of NLuc-S constructs. SecNL-RBD was exemplarily tested for sensitivity against anti-S positive human serum in different concentrations **(a).** An anti-S negative serum pool (NSP) served as control. Construct in sample buffer was incubated without human serum (RBD), with PSP diluted as indicated, and with NSP diluted 1:50 for 30 min at 37°C each, followed by heating of aliquots from 40°C to 70°C in 3°C steps, respectively. Calculation of the first derivative of melting curves resulted in melting peaks **(b).** The shift in apparent Tm is exemplarily indicated for secNL-RBD incubated with PSP diluted 1:20 or in the absence of antibodies. Means ± SD from n=3 independent experiments are shown.

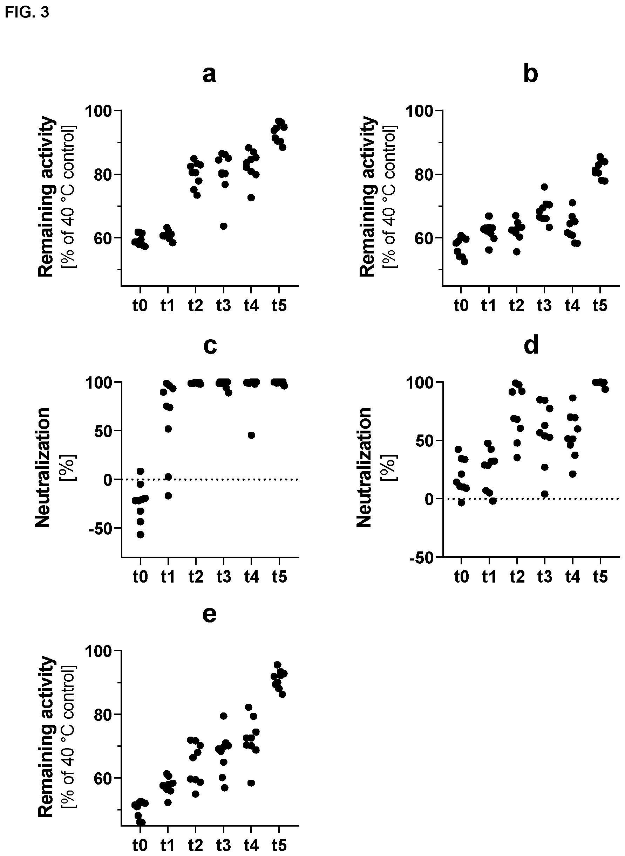

**Figure 3****:** Antibody detection in human serum samples by two-point thermal shift measurements, **a** wt RBD, **b** RBD omicron BA.1 **e** NTD. Constructs were incubated for 30 min with patient sera in a ratio of 1:50 at 37°C. n=9 patient sera from identical individuals were measured per timepoint (t0-5), t0: immediately prior to vaccination, t1: four weeks after first vaccination, t2: four weeks after second vaccination, t3 and t4: three and six months after second vaccination, t5: four weeks after third vaccination. Values represent remaining activity after heating for 3 min to construct-specific apparent Tm compared to the sample heated to 40°C. As controls secNLuc and constructs in sample dilution buffer were included on every plate and allowed to deviate between >95% and 45-55% remaining activity, respectively. For comparison, detection of neutralising antibodies against wt spike and omicron (BA.1) spike by pVNT are shown in panels **c** and **d,** respectively.

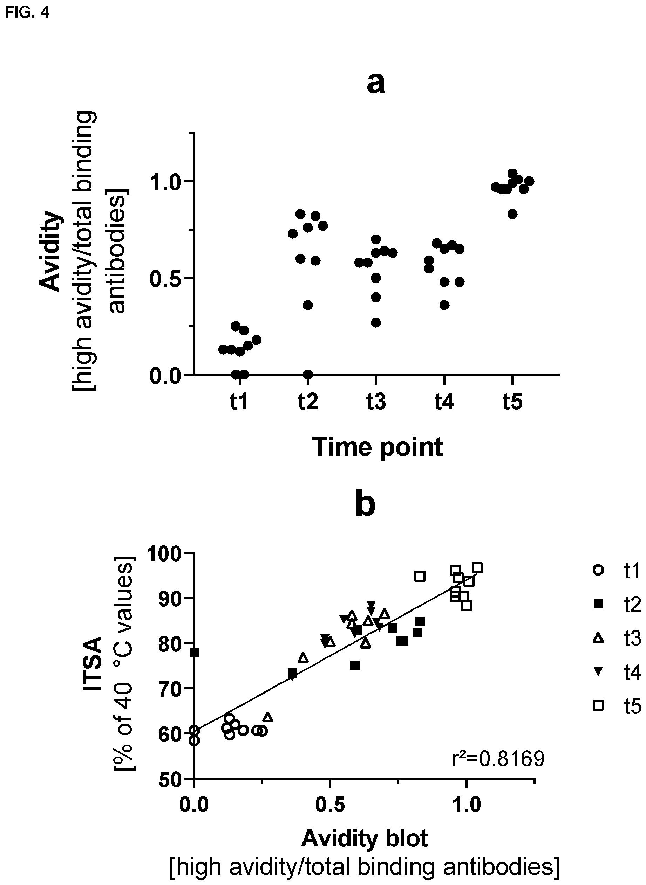

**Figure 4****:** Correlation of RBD ITSA with immunoblot measuring antibody avidity against RBD (Mikrogen recomLine SARS-CoV-2 avidity blot). **(a)** Quantity of high avidity antibodies measured as quotient of urea-treated/untreated serum samples. **(b)** Correlation of the avidity blot results with the values obtained from ITSA measurement for each sample. Statistical analysis was performed by applying linear regression with r²=0.8169.

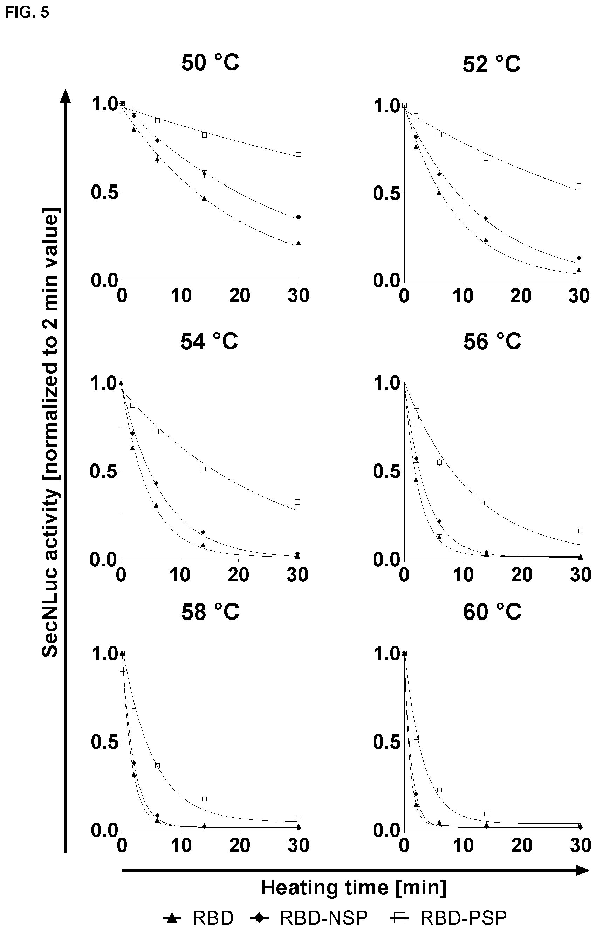

**Figure 5****:** Exponential decay of NLuc activity in secNL-RBD construct incubated with NSP or PSP diluted 1:50 or in the absence of human serum at different temperatures. Normalized exponential decay functions were calculated in GraphPad 8.0 with top value 1 and bottom value 0.

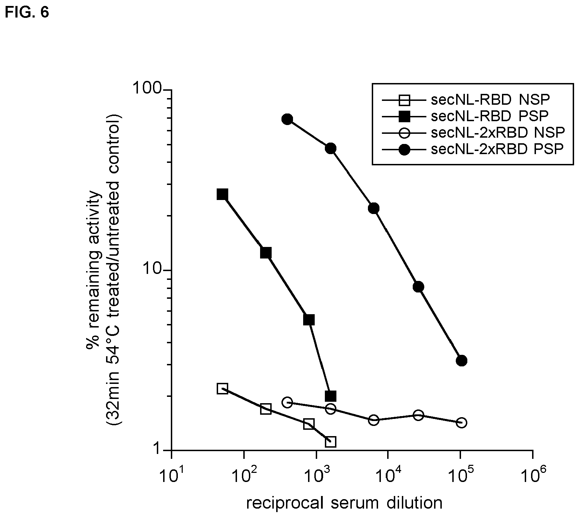

**Figure 6****:** Comparison of constructs containing one and two copies of the RBD, shown exemplarily for secNL-RBD and secNL-2xRBD. Results are indicated as percentage of remaining NLuc activity in samples with thermal treatment (32min at 54°C) as compared to controls without thermal treatment.

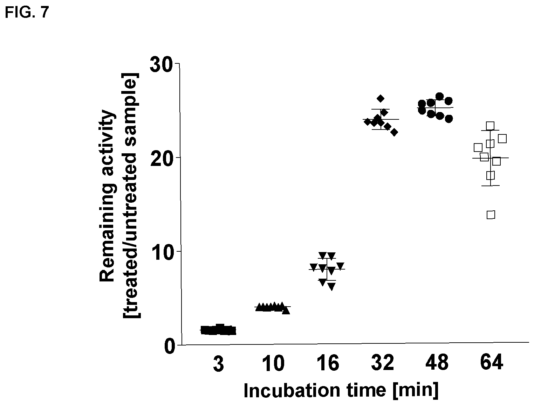

**Figure 7****:** Reproducibility and sensitivity of a quotient calculated from samples treated with PSP 1:50 and untreated samples as diagnostic parameter. n=8 measurement at different incubation times at 54 °C were taken. Remaining NLuc activity of serum-treated sample was divided by activity of untreated sample.

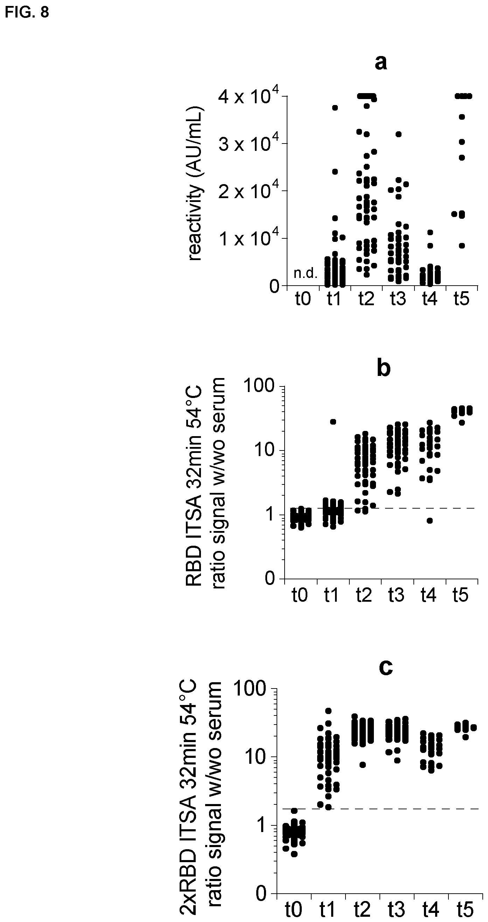

**Figure 8****:** Reactivity of a larger set of sera before and after vaccinations. **a** Reactivity of sera (n=239 vaccinees) in anti-RBD CMIA (gold standard). **b** Reactivity of sera in RBD ITSA (diluted 1:50, 32min 54°C heat treatment). c Reactivity of sera in 2xRBD ITSA (diluted 1:50, 32min 54°C heat treatment). Cut-offs that allow a distinction between vaccinated and unvaccinated individuals with a specificity of 100% in RBD ITSA and 2xRBD ITSA, respectively, are indicated by dashed lines.

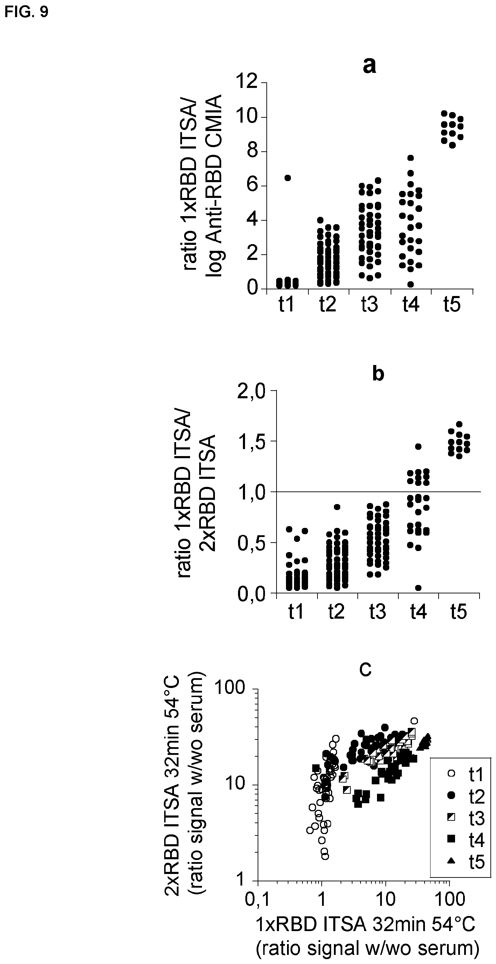

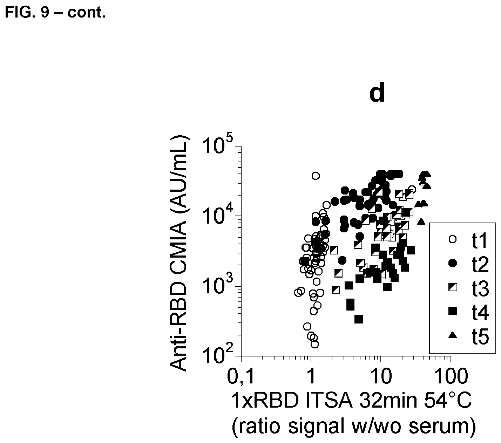

**Figure 9****:** Parallel testing in quantitative seroassay and RBD ITSA. To normalize the result of RBD ITSA to the quantity of anti-RBD antibodies, the RBD ITSA results were divided by either log₁₀ anti-RBD CMIA results **(a)** or results of 2xRBD ITSA **(b).** The distribution of values obtained in RBD ITSA and anti-RBD CMIA **(c)** or 2xRBD ITSA **(d)** are depicted in a scatter plot with indicated time points.

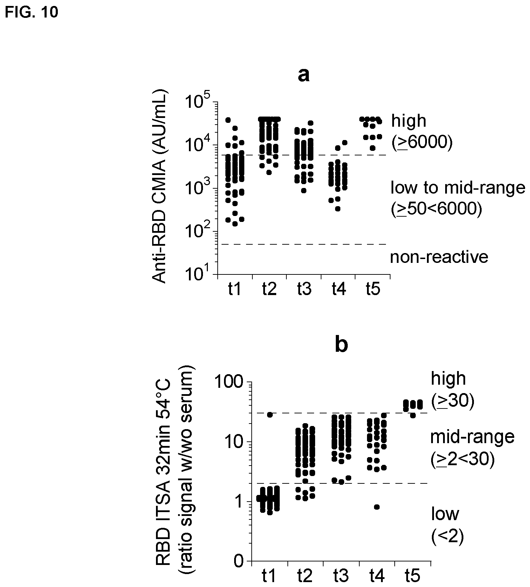

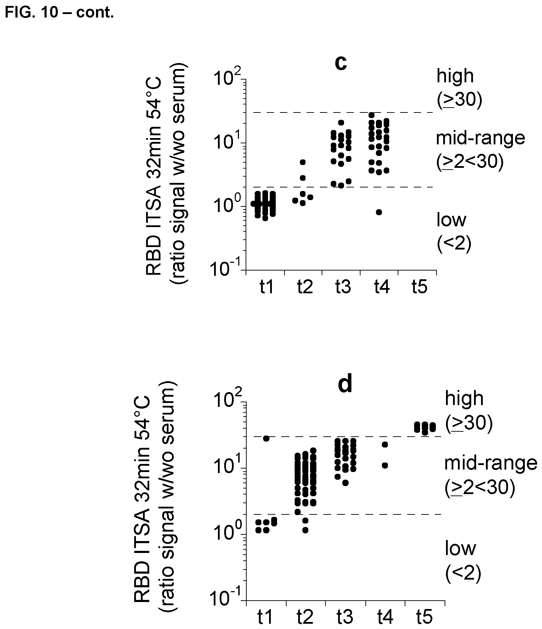

**Figure 10****:** Determination of suitable thresholds in orthogonal testing with Anti-RBD CMIA as first assay and RBD ITSA as second complementary assay to separate sera by number of vaccinations and respective avidity. Thresholds for anti-RBD CMIA **(a)** at <50 and ≥6000 AU/mL, and RBD ITSA **(b)** at <2 and ≥30 are indicated by dotted lines. The differentiation of serum responses by considering antibody level and avidity is depicted in **c** and **d,** in which sera with ≥50-5999 AU/mL (c) or ≥6000 AU/mL **(d)** are represented by their respective RBD ITSA values sorted by time point.

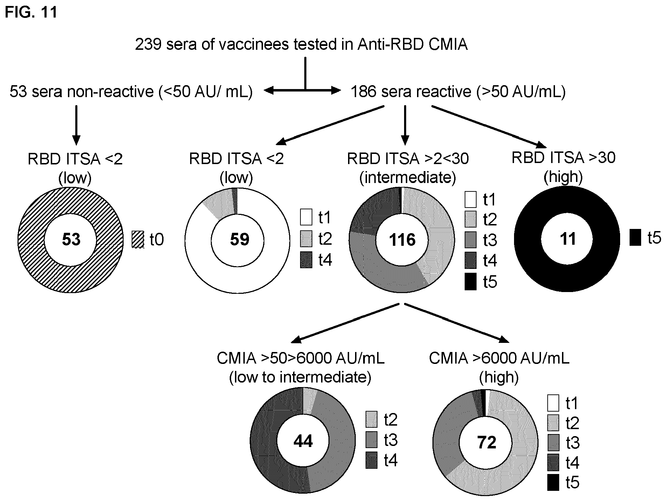

**Figure 11****:** Scheme of sequential testing in anti-RBD CMIA and RBD ITSA resulting in improved diagnostic assessment of the immune response in vaccinees.

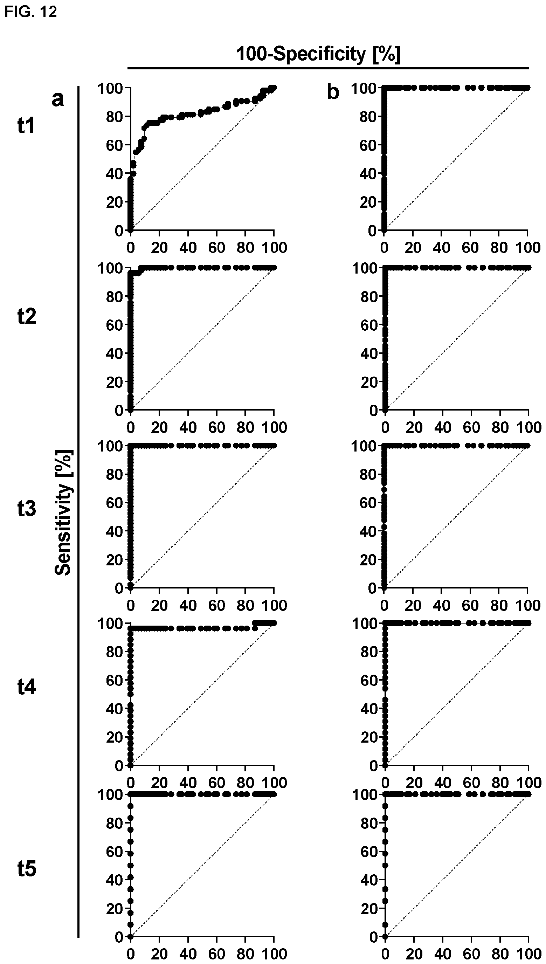

**Figure 12****:** ROC curves for 1xRBD (a) and 2xRBD ITSA **(b)** for each time point compared to the t0 samples. Resulting sensitivity and specificity are given in **tables 3** and **4.**

### DETAILED DESCRIPTION OF THE INVENTION/DETAILED DESCRIPTION OF PREFERRED EMBODIMENTS

A first subject-matter of the invention is directed to a method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii), wherein the ratio of the detected signal (i) and (ii) allows determining the binding characteristic between said antibody and said target polypeptide, and thereby the immune status of the subject is determined.

Preferably, it is foreseen that a value of the ratio of the detected signal above a threshold is indicative of the production of the antibody as a result of a contact with the target polypeptide.

According to the invention, it is foreseen that a threshold is determined which allows determining a value of the ratio of the detected signal which is preferably above a background level, wherein the background level is given in form of a suitable control.

In a preferred embodiment of the invention, it is foreseen that the value of the ratio is in the range from 1 to 10000, preferably from 1.5 to 1000, further preferred from 1.5 to 500, in particular preferred also preferred from 1.5 to 100.

It is further preferred according to the invention that a value of the ratio in the range of less than 2 is indicative of antibodies which bind at a low level; that a value of the ratio in the range of equal or superior to 2 to equal or less than 30 is indicative of antibodies which bind at a mid-range level; and that a value of the ratio in the range of equal or superior to 30 is indicative of antibodies which bind at a high level.

Thus, the method of the present invention allows a distinct characterization of the binding capability of an antibody. Accordingly, it is possible to identify, to characterize, and to determine antibodies concerning their intensity of the binding to the target polypeptide.

It is preferably foreseen that the method of the present invention is (i) indicative of the immune status of a subject with a first contact with the target polypeptide at a first point in time t1, is (ii) indicative of the immune status of a subject with a second contact with the target polypeptide at a second, third, or fourth point in time t2, t3, and/or t4, and is (iii) indicative of the immune status of a subject with a third contact with the target polypeptide at a fifth point in time t5.

Thus, the method of the present invention allows determining the immune status of a subject, which subject received a vaccination, by determining the binding characteristics of the antibodies involved in said vaccination.

Preferably, t1 is four weeks after a first vaccination, t2 is four weeks after the second vaccination, t3 is three months after the second vaccination, t4 is six months after the second vaccination, and t5 is four weeks after the third vaccination.

In a preferred embodiment of the present invention, the antibody is a full-length antibody or an antibody fragment of human or animal origin, and is selected from polyclonal antibodies, monoclonal antibodies, nanobodies and antibody fragments such as Fab- or F(ab)₂-fragment.

In a preferred embodiment of the present invention, determining the immune status comprises determining one or more characteristic of the antibody or antibody fragment selected from avidity, neutralizing capacity, concentration, titre, affinity, maturation, stability, preferably thermal stability, epitope mapping, and paratope mapping.

In further preferred embodiment of the invention, the antibody is derived from active immunisation or from passive immune transfer.

Preferably, the target polypeptide is a monomer or a multimer of the target polypeptide, wherein the multimer comprises monomeric units of one or more variant-specific peptide sequences.

In a further preferred embodiment of the invention, the target polypeptide is selected from the group of a viral polypeptide, bacterial polypeptide, fungal polypeptide, parasite polypeptide, allergenic polypeptides, synthetic polypeptides endogenous antigenic polypeptides such as peptide hormones, cytokines and polypeptides associated with tumors, degenerative, and autoimmune disease.

Preferably, the viral polypeptide derives from the group selected from dsDNA viruses, ssDNA viruses, dsRNA viruses, (+)ssRNA viruses, (-)ssRNA viruses, ssRNA-RT viruses, and dsDNA-RT viruses.

In a preferred embodiment of the invention, the viral polypeptide is a (+)ssRNA virus-derived polypeptide, preferably a coronavirus polypeptide, preferably a SARS-CoV-2 polypeptide, preferably selected from SARS-CoV-2 spike protein, SARS-CoV-2 envelope protein, SARS-CoV-2 membrane protein, and SARS-CoV-2 nucleocapsid protein.

It is further preferred that the viral polypeptide is the RBD domain of the SARS-CoV-2 spike protein, preferably a single or double-RBD domain.

Preferably, the target polypeptide is derived from a viral, bacterial, fungal or parasite polypeptides associated with an acute, chronic, persistent or anamnestic viral, bacterial, fungal or parasite infection in a subject, such as coronavirus infection, cytomegalovirus infection, parvovirus B19 infection, influenza virus infection, hepatitis virus infection, borreliosis or toxoplasmosis.

Accordingly, the method of the present invention is preferably suitable for determining the immune status of a subject concerning vaccination, and in particular determining the success of a vaccination. Preferably, the method of the present invention is also suitable for determining the immune status of a subject in view of a viral or bacterial infection, in particular in view of determining different stages of such an infection. Further, the method of the present invention is also suitable for determining auto-antibodies in view of an autoimmune disease, or determining antibodies in view of allergies.

Preferably, in the method of the present invention it is foreseen that the thermal shift assay comprises the steps of (a) providing a fusion protein of the target polypeptide and a labeling peptide and providing a sample comprising at least one ligand of interest; (b) incubating a liquid mixture comprising the fusion protein and the sample of step (a) under conditions allowing binding of the fusion protein to the sample; (c) incubating the liquid mixture of step (b) under suitable conditions to allow differential denaturation of the fusion protein to the sample and fusion protein not bound to the sample; (d) generating a signal from the fusion protein subjected to the denaturation of step (c) by adding to the mixture a second label suitable to react with the labeling peptide in the fusion protein; (e) detecting the signal in step (d) and the signal of a control comprising the steps (a) to (d) without providing the sample in step (a).

It is further preferred that step (b) comprises incubating at a temperature of 20 to 60°C for 1 to 70 min, preferably a temperature of 54°C for 30 min to 60 min.

Preferably, it is foreseen that step (c) comprises heating the liquid mixture and at least one cooling step, wherein the heating is conducted at about 40°C to about 70°C.

In a further preferred embodiment, the denaturation is a step of heating the liquid mixture to a temperature of about 45°C to about 65°C, preferably of about 50°C to 60°C for a time -period corresponding to 0.5 to 20 apparent half-life times of the antigen in the absence of ligand at the respective temperature.

It is further preferred that step (c) comprises a single isothermal heating step of the sample at a different temperature compared to a control.

It is further preferred that step (c) comprises a single non-isothermal heating step or several isothermal or non-isothermal heating steps interrupted by cooling steps.

It is further preferred that the sample is diluted with a ratio preferably of about 1:20, 1:50, 1:100.

It is further preferred that the sample is applied in a limiting dilution enabling determination of the limit of detection, preferably of about 1:10 to 1:1 000 000.

Preferably, the labeling peptide is an enzyme, a fluorescent peptide, a DNA probe or other biological marker, wherein the enzyme is preferably a luciferase or phosphatase.

Preferably, the method comprises additionally before step (a) and/or after step (e) conducting a different further method for determining the immune status of a subject. Preferably, the further method for determining the immune status of a subject comprises one or more solid-phase immune assay such as Western Blot or ELISA.

Accordingly, in a preferred embodiment of the invention, it is foreseen that the method of the present invention can be combined with a method of the prior art, such as a standard method, for example ELISA.

A second subject-matter of the present invention is directed to the use of the method of the present invention for determining one or more of the following antibody maturation, vaccination status, antibody determination in acute, chronic or anamnestic infection, antibody effectiveness, and/or antibody avidity.

It is further preferred that the method shall be used for screening of antibodies, antibody fragments and/or immunoreceptors specific for a target polypeptide.

A second subject-matter of the present invention is directed to the use of the method of the invention for determining one or more of the following antibody maturation, vaccination status, antibody determination in acute, chronic or anamnestic infection, antibody effectiveness, antibody affinity and/or antibody avidity.

In a further preferred embodiment of the invention, the use of the method of the invention is for screening of antibodies, antibody fragments and/or immunoreceptors specific for a target polypeptide.

A third subject-matter of the present invention is directed to a kit comprising a mixture comprising a fusion protein of a target peptide and a labeling peptide for use in determining the binding property of an antibody for the target peptide.

In a preferred embodiment, it is foreseen that the kit shall be used for conducting the method of the present invention comprising any of the embodiments of the method as described herein.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

All publications and patents cited in this disclosure are incorporated by reference in their entirety. To the extent the material incorporated by reference contradicts or is inconsistent with this specification, the specification will supersede any such material.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of" and "consisting of' may be replaced with either of the other two terms.

### EXAMPLES

The following examples illustrate the invention. These examples should not be construed as to limit the scope of this invention. The examples are included for purposes of illustration and the present invention is limited only by the claims.

### Example 1: Material and Methods

### Cloning of pEN-secNL spike protein constructs

Proteins fused to secNLuc were created using pEN-secNL as backbone vector [34] (Classen, N.; Ulrich, D.; Hofemeier, A.; Hennies, M.T.; Hafezi, W.; Pettke, A.; Romberg, M.-L.; Lorentzen, E.U.; Hensel, A.; Kühn, J.E. Broadly Applicable, Virus-Free Dual Reporter Assay to Identify Compounds Interfering with Membrane Fusion: Performance for HSV-1 and SARS-CoV-2. Viruses 2022, 1354, doi:10.3390/v14071354.) and inserting sequences of different length encoding the SARS-CoV-2 S protein to the C-terminal end of secNLuc as described in Schoefbaenker et al. (2023) [35] (Schoefbaenker, M.; Neddermeyer, R.; Guenther, T.; Mueller, M. M.; Romberg, M.-L.; Classen, N.; Hennies, M. T.; Hrincius, E. R.; Ludwig, S.; Kuehn, J. E.; Lorentzen, E. U. Surrogate Virus Neutralisation Test Based on Nanoluciferase-Tagged Antigens to Quantify Inhibitory Antibodies against SARS-CoV-2 and Characterise Omicron-Specific Reactivity in a Vaccination Cohort. Vaccines 2023, 11(12), 1832, doi:10.3390/vaccines11121832.). Inserts were amplified from pCG1-SARS-2-S [36] (Hoffmann, M.; Kleine-Weber, H.; Schroeder, S.; Krüger, N.; Herrler, T.; Erichsen, S.; Schiergens, T.S.; Herrler, G.; Wu, N.H.; Nitsche, A.; et al. SARS-CoV-2 Cell Entry Depends on ACE2 and TMPRSS2 and Is Blocked by a Clinically Proven Protease Inhibitor. Cell 2020, 181, 271-280.e8, doi:10.1016/j.cell.2020.02.052.) with Q5 high-fidelity DNA polymerase (New England Biolabs, Frankfurt, Germany) after linearization with Sall-HF except for RBD-omicron (BA.1), which was amplified from pCDNA3.1-SARS-CoV-2-omicron containing the S protein of the BA.1 variant. Primers for amplification of RBD sequences were as disclosed in [35] (Schoefbaenker, M.; Neddermeyer, R.; Guenther, T.; Mueller, M. M.; Romberg, M.-L.; Classen, N.; Hennies, M. T.; Hrincius, E. R.; Ludwig, S.; Kuehn, J. E.; Lorentzen, E. U. Surrogate Virus Neutralisation Test Based on Nanoluciferase-Tagged Antigens to Quantify Inhibitory Antibodies against SARS-CoV-2 and Characterise Omicron-Specific Reactivity in a Vaccination Cohort. Vaccines 2023, 11(12), 1832, doi:10.3390/vaccines11121832.). Following digestion of pEN-secNL with BamHl and Notl, constructs were inserted at the C-terminus of the NLuc sequence by In-Fusion cloning (Takara Bio). For pEN-secNL-2xRBD plasmid, pEN-secNL-RBD was opened at the BamHl site and an additional copy of the RBD sequence was amplified with fwd primer 5'-C ATT CTG GCG GGA TCC CGG GTG CAG CCC ACC GAA TCC ATC-3' - SEQ ID NO:1, and bwd primer 5'- G CTG CAC CCG GGA TCC GAA GTT CAC GCA TTT GTT CTT CAC GAG-3' - SEQ ID NO:2 and inserted by In-Fusion cloning. After In-Fusion cloning, constructs were transformed into 10-beta competent E. coli (high efficiency, New England Biolabs). Purified plasmids were obtained from overnight cultures with a Qiaprep Spin Miniprep kit (Qiagen, Hilden, Germany). Sequencing of plasmids was carried out by Eurofins, Ebersberg, Germany. All restriction enzymes were obtained from New England Biolabs.

### Cell culture and protein preparation

BHK cells were cultivated in MEM (Sigma Aldrich, Steinheim, Germany) containing 10% FCS, 1% penicillin/streptomycin solution (10,000 U/10,000 mg/mL, Merck, Darmstadt, Germany), 1% NEAA solution 100x (Sigma-Aldrich) and 1% L-glutamine solution 200 mM (Sigma-Aldrich) in 75 cm² culture flasks at 37 °C/5% CO₂. For transfection of plasmids, cells were washed with PBS once, detached with trypsin/EDTA solution, resuspended in medium and counted. A quantity of 2 or 6×10⁵ cells was seeded into each well of 24- or 6-well culture plates, respectively, and incubated for 20 h. Plasmids (pEN-secNL-RBD, pEN-secNL-2xRBD, pEN-secNL-RBD-omicron, pEN-secNL-NTD, pEN-secNL-S15-541: 200 ng/24-well; pEN-secNL-spike-CSdel-PPmut: 2 µg/6-well) were mixed with 1.5 µl (24-well plates) or 4 µL (6-well plates) lipofectamine and cells were incubated with transfection mix for 3.5 h. After washing with PBS once, cells were incubated for 24-48 h. Supernatants were collected, pooled, and centrifuged for 10 min at 3500 g, subsequently aliquoted and stored at -20 °C. Protein quantity and function was analyzed by measuring NLuc activity, designated as rlu/mL, and ability to bind to ACE2-coated plates after one freeze-thaw cycle.

### Thermal shift analysis

Unless otherwise indicated, for preparation of each individual reaction an amount of fusion protein containing supernatant corresponding to 5×10⁵ rlu was diluted in assay buffer (87.6 g NaCl, 26 g Tris-HCl, 4.24 g Tris, 0.05% Tween 20, ad 1000 mL with bidistilled water) to a final volume of 100 µL. For serum analysis, 2 µL of serum per 100 µL was added and samples were incubated for 30 min at 37 °C. 100 µL of the sample were transferred to PCR tubes. Heating of samples was carried out in a TC20 thermocycler (Bio-Rad, Hercules, US) for the indicated incubation time and temperatures. After heating samples were stored on ice, and 20 µL of each sample was transferred to a black 96-well culture plate. 80 µL of distilled water was added per well, and 20 µL of NanoGlo reagent (Promega, Walldorf, Germany) containing 1:50 substrate/lysis buffer was quickly pipetted into the wells, according to the manufacturer's instructions. After 3 min of incubation, luminescence was measured in a Glomax Explorer luminometer (Promega) with an integration time of 0.3 sec.

### Avidity measurement

For determination of antibody avidity in human sera the recomLine SARS-CoV-2 IgG [Avidität] kit (Mikrogen, Neuried, Germany) was applied. The protocol was carried out according to the manufacturer's instruction. Quantitative results were obtained from colorimetric readout of the blot strips. Results were expressed as quotient between the entire and high avidity IgG antibody reactivity in the sera.

### Anti-SARS-CoV-2 IgG quantification

Quantification of anti-SARS-CoV-2 S IgG antibodies from single patient sera and serum pools were performed using the SARS-CoV_2 II Quant Assay on an Architect i1000SR system (Abbott, Illinois, US), according to the manufacturer's manual.

### pVNT

A pseudovirus-based virus neutralization test (pVNT) was performed as described in Schoefbaenker et al. (2023) [35] (Schoefbaenker, M.; Neddermeyer, R.; Guenther, T.; Mueller, M. M.; Romberg, M.-L.; Classen, N.; Hennies, M. T.; Hrincius, E. R.; Ludwig, S.; Kuehn, J. E.; Lorentzen, E. U. Surrogate Virus Neutralisation Test Based on Nanoluciferase-Tagged Antigens to Quantify Inhibitory Antibodies against SARS-CoV-2 and Characterise Omicron-Specific Reactivity in a Vaccination Cohort. Vaccines 2023, 11(12), 1832, doi:10.3390/vaccines11121832.). The samples were tested using plasmids for wt S (pCG1-SARS-2-S) or the omicron BA.1 variant S (pcDNA3.1 SARS-2 Omicron). The cut-off to distinguish samples from samples at t0 with 100% specificity was defined as ≥65% inhibition.

### Human serum samples

The human serum samples were collected at different time points from initially SARS-CoV-2 S and N antibody negative subjects before and after first, second and third vaccination, respectively. The positive serum pool (PSP) was pooled from sera collected at t2, e.g. four weeks after the second vaccination, representing antibodies of intermediate avidity. The negative serum pool (NSP) was pooled from sera collected before vaccination. The trial was approved by the ethics committee of the Ärztekammer Westfalen-Lippe and the University of Münster (2021-039-f-S).

### Statistics

Data analysis was carried out using GraphPad Prism 8.0 software. Standard deviations were calculated from at least n = 3 independent experiments if not stated otherwise. Curve fit and determination of Tm values was performed by using non-linear regression/Boltzmann sigmoidal function. Significance was calculated by either student's t-test for comparison of two groups, or one-way ANOVA test, followed by Tukey's post-test in case of significant difference for comparison of more than two groups. *** means highly significant (p<0.001), ** means very significant (p<0.01) and * means significant (p<0.05).

### Example 2: Melting properties of NLuc-tagged spike constructs

Determination of the apparent melting temperature (Tm) for each construct was carried out by taking measurements of single samples in 3 °C steps for 3 min, respectively. Values were normalized to 40 °C value as 100% and 0 rlu was set to 0%. According to Dart et al. [22] (Dart, M.L.; Machleidt, T.; Jost, E.; Schwinn, M.K.; Robers, M.B.; Shi, C.; Kirkland, T.A.; Killoran, M.P.; Wilkinson, J.M.; Hartnett, J.R.; et al. Homogeneous Assay for Target Engagement Utilizing Bioluminescent Thermal Shift. ACS Med. Chem. Lett. 2018, 9, 546-551, doi:10.1021/acsmedchemlett.8b00081.), NLuc itself reveals a melting temperature above 70 °C, indicating that melting of the luciferase domain does not significantly take influence on constructs beneath 60 °C. All constructs showed an individual melting curve correlating with their biochemical properties **(****Figure 1a****-e).** The short constructs secNL-RBD and secNL-NTD comprised the lowest deviation from the calculated standard curve with R² values above 0.99, compared to secNL-S, which with an R² of 0.9698 showed a weaker sigmoidal correlation and higher deviation between single experiments. This may indicate the better suitability of short protein fragments for ITSA compared to large, multi-domain proteins. SecNL-RBD-omicron (BA.1) revealed a slightly higher apparent melting temperature as compared to secNL-RBD. Calculation of the first derivative of the melting curves resulted in melting peaks of the constructs, enabling to define melting points and the evaluation of suitability of a construct for use in ITSA **(****Figure 1f****).** The peak of the secNL-S protein was broader than those of the short constructs, probably indicating more complex melting properties. Furthermore, the Tm was found to be very high (61 °C). The construct was thus assessed as less suitable for ITSA development and was not further investigated.

### Example 3: Impact of human serum antibodies on melting behavior of NLuc-tagged spike antigens

The assay principle of reporter enzyme-linked TSA is, compared to common dye dependent TSA/DSF approaches, insensitive to the presence of accompanying proteins in the measured samples. Hence, it was assumed that binding of SARS-CoV-2 S-reactive antibodies present in human sera to the NLuc-tagged diagnostic antigens could have a specific impact on the melting behavior of the constructs without affecting the read-out of the assay non-specifically, e.g., due to accompanying serum proteins. A pool from anti-S positive sera (PSP) obtained from 17 two-fold vaccinated individuals without SARS-CoV-2 infection [35] (Schoefbaenker, M.; Neddermeyer, R.; Guenther, T.; Mueller, M. M.; Romberg, M.-L.; Classen, N.; Hennies, M. T.; Hrincius, E. R.; Ludwig, S.; Kuehn, J. E.; Lorentzen, E. U. Surrogate Virus Neutralisation Test Based on Nanoluciferase-Tagged Antigens to Quantify Inhibitory Antibodies against SARS-CoV-2 and Characterise Omicron-Specific Reactivity in a Vaccination Cohort. Vaccines 2023, 11(12), 1832, doi:10.3390/vaccines11121832.) was added to the construct solution in different concentrations to determine detection limit and saturation concentration. Incubation of secNLuc-tagged antigens for 30 min with human serum before measurement at different temperatures resulted in a temperature and serum dilution dependent shift of the apparent melting temperature for all constructs, exemplarily shown for secNL-RBD **(****Figure 2****).** In particular, secNL-RBD but also secNL-S15-541 and secNL-NTD (data not shown) were sensitive to the presence of serum.

The melting temperatures were compared, and significance of differences was calculated as given in **Table 1.** At serum dilutions of 1:100 and lower, the maximal effect was achieved, resulting in a thermal shift of about 4.5 °C, and indicating a specific interaction of serum antibodies with the antigen construct. The thermal shift was found to be significant compared to samples containing only construct up to a serum dilution of 1:100. Calculation of the first derivative of the melting curve, the shift of the melting peak was clearly visible and concentration dependent **(****Figure 2b****).** Addition of negative serum pool (NSP, identical donors as for the PSP, sera obtained before first vaccination) diluted 1:50 did not result in significant thermal shift compared to serum-free control and differed significantly from PSP in a dilution up to 1:100 **(Table 1).**

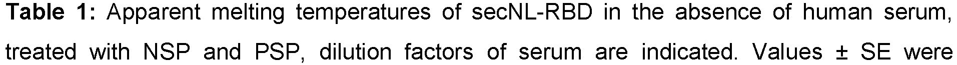

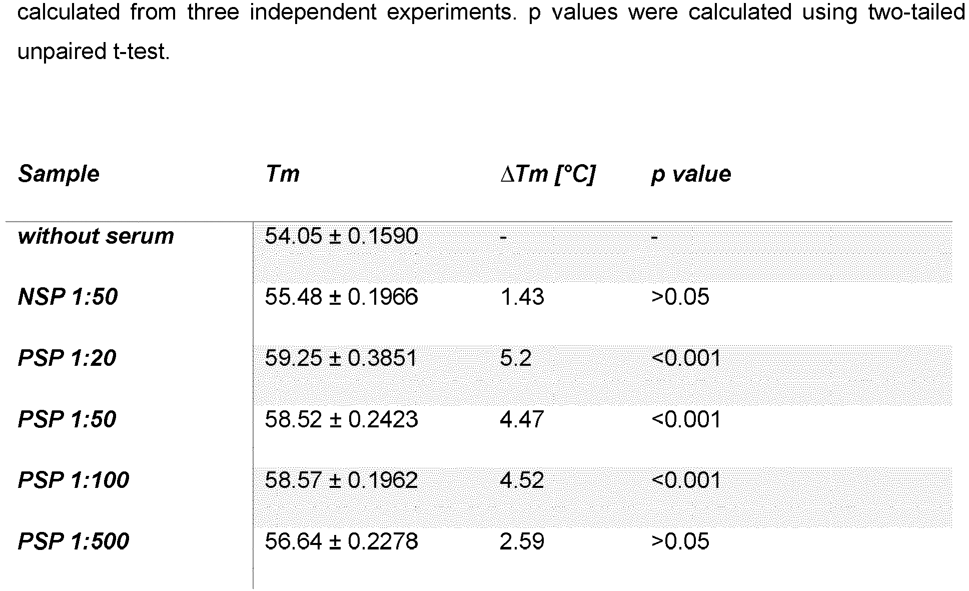

### Example 4: Detection of antibody response in serially taken serum samples of vaccinees by ITSA

To determine the ability of the assay to detect immune responses to infection or vaccination, human serum samples taken from nine vaccinees before first vaccination (t0), four weeks after first vaccination (t1), four weeks after second vaccination (t2), three (t3) and six months (t4) after second vaccination and four weeks after third vaccination (t5) against SARS-CoV-2, respectively, were tested. For that purpose, the assay principle was reduced to a two-point measurement setup comparing the remaining activity of serum and heat-treated samples to a control sample without heat treatment. Constructs were incubated with serum samples at a dilution of 1:50 in assay buffer for 30 min before samples were heated to either 40 °C or apparent Tm (54°C for secNL-RBD) as determined in the former experiments. An increase in remaining activity after heating was rated to indicate the interaction of serum sample with the NLuc-S construct. As parameters for a valid measurement reaction, controls containing construct diluted in sample dilution buffer in the absence of serum or NLuc only were included. After temperature treatment for 3 min at 54 °C, these controls were allowed to range between 45 and 55% or >95% of the control samples without heat treatment, respectively. Sera tested showed vaccine-dose dependent effects on secNL-RBD **(****Figure 3a****),** secNL-RBD-omicron **(****Figure 3b****)** and secNL-NTD **(****Figure 3e****).** Namely, no construct stabilization was observed before vaccination, and an increasing reactivity was detected after vaccination, reaching a maximum after the third vaccination, and remaining stable for six months after second vaccination. The results correlated well with the results of a pVNT as gold standard, performed with the same samples for wt and omicron BA.1 S **(****Figure 3c**,d). SecNL-RBD-omicron (BA.1) was the only construct comprising alterations in the protein sequence distinct from the wt SARS-CoV-2 S protein, which leads to diminished immunological response to vaccination *in vivo* and to a decreased susceptibility of therapeutic monoclonal antibodies [37,38] (Carreño, J.M.; Alshammary, H.; Tcheou, J.; Singh, G.; Raskin, A.J.; Kawabata, H.; Sominsky, L.A.; Clark, J.J.; Adelsberg, D.C.; Bielak, D.A.; et al. Activity of convalescent and vaccine serum against SARS-CoV-2 Omicron. Nature 2022, 602, 682-688, doi:10.1038/s41586-022-04399-5.; Hoffmann, M.; Krüger, N.; Schulz, S.; Cossmann, A.; Rocha, C.; Kempf, A.; Nehlmeier, I.; Graichen, L.; Moldenhauer, A.S.; Winkler, M.S.; et al. The Omicron variant is highly resistant against antibody-mediated neutralization: Implications for control of the COVID-19 pandemic. Cell 2022, 185, 447-456.e11, doi:10.1016/j.cell.2021.12.032.). As compared to secNL-RBD, the RBD omicron BA.1 construct showed reduced reactivity with the sera of individuals vaccinated with wt S during time-points t2 to t5, suggesting the ability of the assay to detect variant-specific differences in antibody responses to the RBD **(****Figure 3****).** Variant-specific effects in reactivity of sera were also observed in pVNT.

### Example 5: Effect of antibody avidity on reactivity in ITSA

In the former experiments, an interaction of the constructs with human antibodies, resulting in a thermal stabilization of the constructs, was evident. To categorize the type of interaction, the results were correlated with the results obtained in an commercial assay [39] (Bauer, G.; Struck, F.; Schreiner, P.; Staschik, E.; Soutschek, E.; & Motz, M. The challenge of avidity determination in SARS-CoV-2 serology. Journal of Medical Virology 2021, 93(5), 3092-3104, doi:10.1002/jmv.26863.) measuring antibody avidity in serum samples **(****Figure 4a****).** Linear regression revealed good overall correlation between both assays as well as differences for a few samples **(****Figure 4b****),** indicating that multiple factors may have an impact on the outcome of either the commercial avidity assay or ITSA. In general, it can be deduced from this experiment, that the assay does distinguish between high and low avidity antibodies.

### Example 6: Optimization of the assay parameters of RBD ITSA for routine diagnostic purposes

In the following, the impact of incubation temperature and time on outcome of ITSA was analyzed in detail to increase sensitivity and specificity. For that purpose, constructs were incubated with NSP and PSP for 30 min at 37°C and subsequently heated to indicated temperatures for 2, 4, 8, 16 and 32 min. A temperature-dependent exponential decay of the remaining secNLuc activity was evident. Except for a thermal shift to 60 °C, NLuc-activity in samples containing PSP was significantly higher after 32 min of temperature treatment than in control samples **(****Figure 5****).** The exponential decay of secNLuc activity during heating allowed determination of half-lifes (t_{1/2}). At 54°C, the biggest difference of remaining NLuc activity between PSP-treated and NSP-treated or untreated samples, respectively, was detected after approximately 15 min. Heat treatment of secNL-RBD in the absence of antibodies for 32 min at 54°C (corresponding to approximately 10 half-lifes of the construct) decreased NLuc-activity almost to background levels, whereas PSP-treated samples still showed high NLuc activity. Incubation of samples for 32 min at 54°C was therefore considered to be well suited for determination of antibody reactivity with secNL-RBD in ITSA.

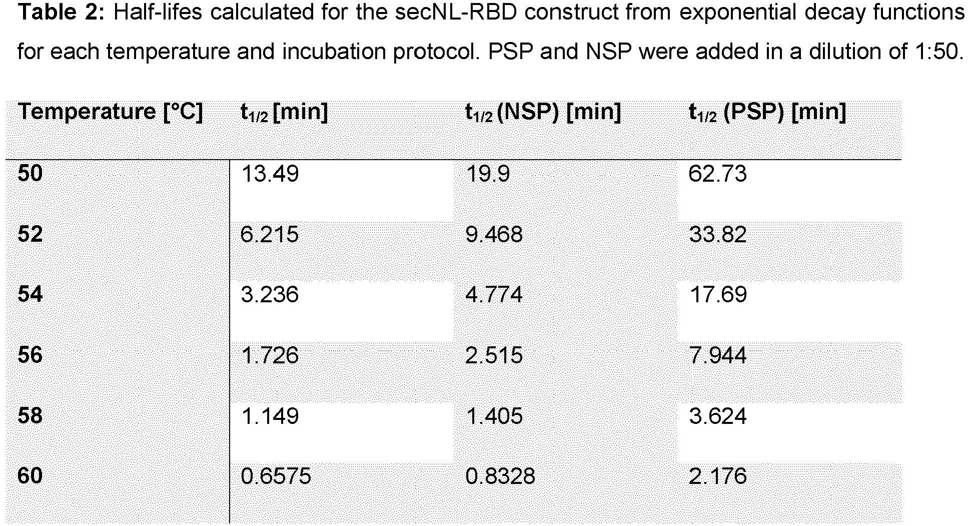

A double antigen construct comprising two copies of the wt RBD sequence arranged in tandem (pEN-secNL-2xRBD) was created to analyze whether bivalent binding of antibodies may further enhance avidity and thereby thermal stabilization of constructs and sensitivity of the assay. The apparent Tₘ of the 2x wt RBD construct was determined to be approximately 54 °C, comparable to the 1x wt RBD construct. Using a limiting dilution of PSP, the 2x wt RBD construct resulted in an approximately 100-fold increased sensitivity of ITSA as compared to the single RBD construct and allowed to detect an effect of PSP in ITSA even at a dilution of up to 1:100,000 **(****Figure 6****).**

To apply ITSA as diagnostic tool in serology, read-out parameters must be defined, enabling reproducible and comparable results. Therefore, we determined by eightfold parallel testing the optimal time point of measurement and variation of values to maximize the discrimination of antibody-treated from untreated samples in ITSA **(****Figure 7****).** Using the PSP diluted 1:50 as source of antibodies with intermediate avidity, 32 or 48 min were found as optimal incubation time for an isothermal heating to 54°C. Incubation of secNL-RBD for 32 min at 54°C increased the quotient of NLuc activity of samples with and without addition of serum up to 25-fold and resulted in low intra-assay variation. For every time point, the activity of serum-treated samples differed highly significantly from the untreated samples (p<0.001). After 64 min, precision of the results decreased.

### Example 7: Evaluation of the diagnostic performance of ITSA

Using the optimized assay conditions, a larger set of sera (n = 239) obtained before and after vaccination (mRNA vaccine) of SARS-CoV-2 naive individuals was applied to evaluate the sensitivity and specificity of the assay system. Reactivity of sera in a commercial quantitative anti-RBD chemiluminescence microparticle immunoassay (CMIA, Abbott) served as gold standard **(****Figure 8a****).** Cut-offs to achieve 100% specificity were defined by calculating receiver operating characteristic (ROC) curves **(****Figure 12****)** and comparing reactivity before vaccination (t0, n = 53) with reactivity four weeks after the first (t1, n = 53) and second vaccination (t2, n = 53). A reduced number of sera were also obtained three months (t3, n = 42) and six months (t4, n = 26) after the second vaccination, and four weeks after the third vaccination (t5, n = 12), respectively.

This showed, that ITSA with the single RBD construct has a low sensitivity of 35.85% (19/53 reactive) at t1 **(****Figure 8b****).** At t2, the sensitivity of ITSA with the single RBD construct increased to 96.23% (51/53) reactivity, reached 100% reactivity at t3 and t5, and was 96.15% at t4 (25/26 sera reactive) **(Table 3).** Concerning signal strength, the ratios of antibody-treated to untreated samples were only slightly elevated at t1 as compared to t0, showed a strong rise from t1 to t2, some additional increase from t2 to t3 and remained at elevated levels at t4. In all vaccinees tested, the third vaccination resulted in an additional massive increase in the reactivity ratios (Figure 13).

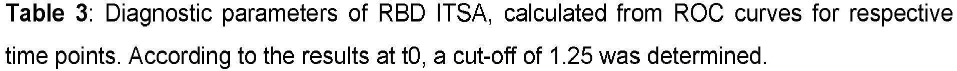

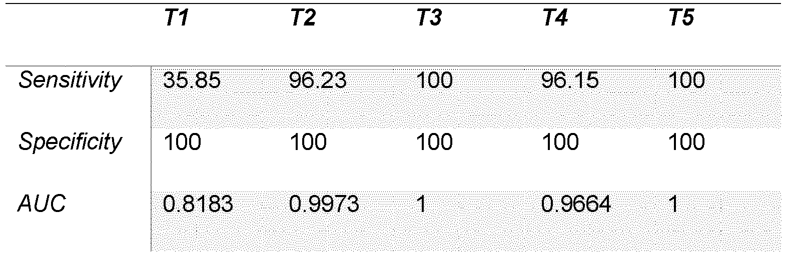

In contrast, ITSA with the double-RBD construct achieved 100% sensitivity at t1, and at all other time points (t2 to t5) **(Table 4).** Reactivity ratios obtained with the double RBD construct in ITSA strongly increased from t0 to t1 and t2, showed some decrease from t3 to t4 and increased again at t5 **(****Figure 8c****),** much alike the results from the quantitative CMIA. Due to its high sensitivity already at t1, the 2xRBD ITSA is therefore suitable for the qualitative detection of antibodies against SARS-CoV-2 S.

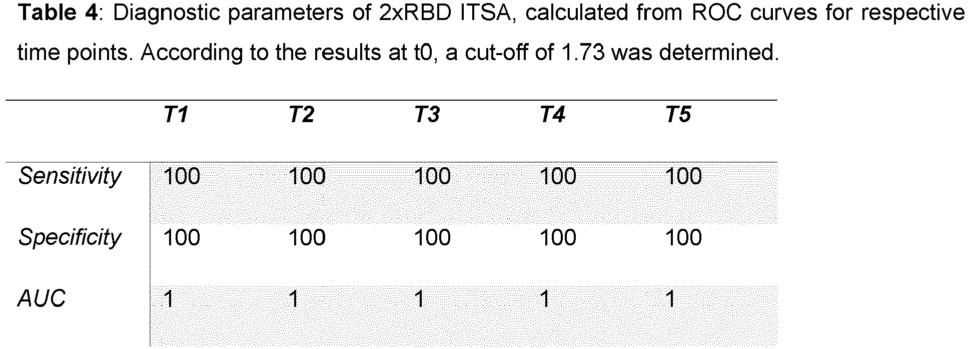

By calculating quotients from the RBD ITSA and a second assay which essentially detects antibody levels (2xRBD ITSA or CMIA), values permanently increasing over time were obtained as to be expected for continuous affinity maturation of antibodies from t1 to t5 **(****Figure 9****).** Particularly by calculating the quotient of RBD/2xRBD ITSA **(****Figure 9b****),** sera with high avidity could be separated from those with low or intermediate avidity. This separation could be visualized by plotting the results of the quantitative test against those of the RBD ITSA in a scatter plot **(****Figure 9c**,d), clearly indicating a trend towards time-dependent, different reaction patterns of sera when considering both parameters.

In the following, cut-off values were introduced for both CMIA and RBD ITSA to optimize the differentiation of the time of blood collection and therefore the development of the immune status of vaccinees **(****Figure 10****).** For the anti-RBD CMIA, according to the manufacturer <50 AU/mL was considered negative. A second threshold was set at ≥6000 AU/mL, indicating a high quantity of anti-RBD IgG which was detected in all serum samples of vaccinees obtained at t5 and most serum samples collected at t2, respectively **(****Figure 10a****).** The lower threshold for the RBD ITSA was set at <2, discriminating samples from t0 (53/53) and t1 (52/53) from the other time points **(****Figure 8b**, 10b). The second threshold was placed at ≥30, by which the vast majority of samples from t5 (11/12) could be discriminated from the remaining samples. By separating the samples according to their values in the anti-RBD CMIA into two groups (≥50 - <6000 and ≥6000 AU/mL, **Figure 10c**,d) and applying the RBD ITSA as second, orthogonal marker, a differentiation of samples by time points and thereby by vaccination and avidity status became possible.

An exemplary evaluation scheme is given in **Figure 11****,** classifying sera first as reactive/nonreactive by CMIA and subsequently as low, intermediate and highly reactive in RBD ITSA. This allowed for correct assignment of 53/53 sera to t0 (before vaccination), of 52/53 sera to t1 (first vaccination, reactive in CMIA, low avidity in RBD ITSA), of 114/121 sera to t2, t3, and t4 (reactive in CMIA, intermediate avidity in RBD ITSA, second vaccination), and of 11/12 sera to t5 (reactive in CMIA, high avidity in RBD ITSA, third vaccination).

If the reaction strength of positive sera in CMIA was used as a further criterion, samples of individuals vaccinated twice could be separated with improved accuracy. Thus, most sera obtained at t2 had high antibody levels in CMIA and intermediate avidity, whereas the majority of sera collected at t4 had low antibody levels in CMIA and intermediate avidity **(****Figure 11****).** Below, classification of sera according to the proposed scheme based on qualitative CMIA results and quantitative RBD ITSA results is listed. This highlights the ability of the assay system to receive relevant additional information on the quality of immune response, e.g., number of antigen contacts and affinity maturation of antibodies.

### Assignment of sera to the number of vaccinations

ITSA <2 + CMIA <50, n = 53: no vaccination, t0 (53/53), other time points: none, identification of t0 sera with 100% sensitivity and 100% specificity
ITSA <2 + CMIA ≥50, n = 59: first vaccination, t1 (52/53), other time points: 7 (6x t2, 1x t4), identification of t1 sera with 98% sensitivity and 88% specificity
ITSA ≥2-30 + CMIA ≥50, n = 116: second vaccination, t2-4 (114/121), other time points: 2 (1x t1, 1x t5), identification of t2, t3, and t4 sera with 94% sensitivity and 98% specificity
ITSA ≥30 + CMIA ≥50, n = 11: third vaccination, t5 (11/12), other time points: none, identification of t5 sera with 92% sensitivity and 100% specificity

Determination of antibody levels is commonly used to characterize the humoral immune response against anamnestic, acute or chronic infections and vaccinations. Antibody quantity only partially reflects functional aspects of antibody responses in vivo. Determination of functional affinity or avidity gives important additional information concerning affinity maturation of antibodies and the resulting strength of antibody/antigen interaction, and can be used to characterize the outcome of vaccination, the time point of infection or immune status in general. Common methods for avidity measurement require elaborated protocols including protein denaturation steps with chaotropic substances like urea. Information on antibody avidity is obtained by comparing the results in treated and untreated serum samples by densitometric scanning or ELISA. With the present invention, the inventors evaluated and adapted thermal shift analysis (TSA) for determination of the immune status in a subject by determining the binding characteristic between an antibody and a target polypeptide. The resulting assay was termed immuno TSA (ITSA). The inventors established an ITSA protocol measuring the influence of human serum antibodies on thermal stability of antigens from SARS-CoV-2 virus S protein, utilizing a modified secreted NanoLuc (secNLuc)-based thermal shift assay. The inventors compared the results with the results of commercial serological assays to examine the mode of action and the value of thermal shift analysis for routine diagnostics. Exemplarily, nanoluciferase-tagged SARS-CoV-2 spike protein fragments were employed as diagnostic antigens which allowed to link the folding status of constructs to chemiluminescence.

The results show that binding of antibodies to diagnostic antigens induces thermal resistance, which can be precisely quantitated by chemiluminescence. At saturating concentrations of antibodies, the extent of thermal resistance directly correlates with antibody avidity. The method of the present invention is able to quantitate the strength of antibody-antigen reactions in fluid phase, thereby greatly facilitating determination of antibody avidity in polyclonal human serum samples. It holds promise as it is homogenous, robust, simple to perform in samples of human and non-human origin, and broadly applicable to diverse scientific and diagnostic questions.

With the present invention, the inventors present an easy-to-use homogenous serological assay for investigation of the SARS-CoV-2-specific immune response. The results of the examples demonstrate that the method of the present invention is suited for investigation of antibody production and maturation in human serum samples. The method of the present invention comprises benefits compared to serological assays commonly used in routine diagnostics. The ability to exclude non- or low-specifically binding antibodies is an advantage compared to, e.g., ELISA. Most commercially available test systems are solid phase based, which alters the binding kinetics of antigen and antibody and complicates establishment of automated workflows. This obstacle can be overcome in ITSA presented here by direct measuring in fluid phase, enabling rapid transfer of the reaction mix from one device to another.

Correlation of commercial serological avidity assays with the results obtained in this study indicate the ability of the method of the present invention - ITSA - to function as useful tool in diagnostics with the benefit of simple, cheap, and quick analysis, probably scalable to 364 well plate and being fully automatable. Additionally, the results insinuate a broad field of applications, for diagnostics may be carried out with a variety of antigens, e.g., of different bacterial and viral species. An interesting field of application in routine diagnostic could be the examination of immune response in high-risk patients as a marker correlated to better outcome and to apply evidence based pharmaceutical treatment, especially the use of monoclonal antibodies. Also, detection and monitoring of autoimmune diseases may be a potential application, particularly due to the assays ability to detect an immune response months after first antigen exposition.

Comparison with a common quantitative ELISA reveals that the method of the present invention - ITSA - is able to detect antibodies showing massively increased avidity for bi- or polyvalent antigens with a sensitivity two or more log orders higher. These results indicate the possibility of distinguishing between intermediate and high avidity antibodies in human serum samples by applying higher antibody dilutions and comparing binding to mono- and bi- or polyvalent antigens. It also indicates that stabilization activity under saturating antibody concentrations is mainly independent from antibody quantity, questioning the outcome of antibody titers as a valid tool in diagnostics after several vaccinations or following infection.

The method of the present invention is also able to deliver valuable results at melting temperatures of 60°C or below because of mutual stabilization of antibody and antigen, which is expected to increase the melting temperature of high affinity antibodies as well. Additionally, melting of non-binding antibodies is not impairing measurement.

Advantageously, the method of the present invention can be modified by varying reporter enzymes or using fluorescent proteins instead of NLuc to increase sensitivity and thermostability of the readout system and to minimize costs and duration.

The invention is also characterized by the following items.
1. A method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii), wherein the ratio of the detected signal (i) and (ii) allows determining the binding characteristic between said antibody and said target polypeptide, and thereby the immune status of the subject is determined.
2. The method of item 1, wherein a value of the ratio above a threshold is indicative of the production of the antibody as a result of a contact with the target polypeptide.
3. The method of item 1 or 2, wherein the value of the ratio is in the range from 1 to 10000, preferably from 1.5 to 1000, further preferred from 1.5 to 500, in particular preferred also preferred from 1.5 to 100.
4. The method of any one of items 1 to 3, wherein
   (i) a value of the ratio in the range of less than 2 is indicative of antibodies which bind at a low level;
   (ii) a value of the ratio in the range of equal or superior to 2 to equal or less than 30 is indicative of antibodies which bind at a mid-range level; and
   (iii) a value of the ratio in the range of equal or superior to 30 is indicative of antibodies which bind at a high level.
5. The method of any one of items 1 to 4, wherein
   (i) is indicative of the immune status of a subject with a first contact with the target polypeptide at a first point in time t1,
   (ii) is indicative of the immune status of a subject with a second contact with the target polypeptide at a second, third, or fourth point in time t2, t3, and/or t4, and
   (iii) is indicative of the immune status of a subject with a third contact with the target polypeptide at a fifth point in time t5.
6. The method of any one of items 1 to 5, wherein the antibody is a full-length antibody or an antibody fragment of human or animal origin, and is selected from polyclonal antibodies, monoclonal antibodies, nanobodies and antibody fragments such as Fab- or F(ab)2-fragment.
7. The method of any one of items 1 to 6, wherein determining the binding characteristic between the antibody and the target polypeptide comprises determining one or more characteristic of the antibody or antibody fragment selected from avidity, neutralizing capacity, concentration, titre, affinity, maturation, stability, preferably thermal stability, epitope mapping, and paratope mapping.
8. The method of any one of items 1 to 7, wherein the antibody is derived from active immunisation or from passive immune transfer.
9. The method of any one of items 1 to 8, wherein the target polypeptide is a monomer or a multimer of the target polypeptide, wherein the multimer comprises monomeric units of one or more variant-specific peptide sequences.
10. The method of any one of items 1 to 9, wherein the target polypeptide is selected from the group of viral polypeptide, bacterial polypeptide, fungal polypeptide, parasite polypeptide, allergenic polypeptides, synthetic polypeptides endogenous antigenic polypeptides such as peptide hormones, cytokines and polypeptides associated with tumors, degenerative or autoimmune disease.
11. The method of any one of items 1 to 10, wherein the viral polypeptide derives from the group selected from dsDNA viruses, ssDNA viruses, dsRNA viruses, (+)ssRNA viruses, (-)ssRNA viruses, ssRNA-RT viruses, and dsDNA-RT viruses.
12. The method of any one of items 1 to 11, wherein the viral polypeptide is a (+)ssRNA virus-derived polypeptide, preferably a coronavirus polypeptide, preferably a SARS-CoV-2 polypeptide, preferably selected from SARS-CoV-2 spike protein, SARS-CoV-2 envelope protein, SARS-CoV-2 membrane protein, and SARS-CoV-2 nucleocapsid protein.
13. The method of any one of items 1 to 12, wherein the viral polypeptide is the RBD domain of the SARS-CoV-2 spike protein, preferably a single or double-RBD domain.
14. The method of any one of items 1 to 13, wherein the target polypeptide is a viral, bacterial, fungal or parasite polypeptides associated with an acute, chronic, persistent or anamnestic viral, bacterial, fungal or parasite infection in a subject, such as coronavirus infection, cytomegalovirus infection, parvovirus B19 infection, influenza virus, hepatitis virus, borreliosis or toxoplasmosis.
15. The method of any one of items 1 to 14, wherein the thermal shift assay comprises the steps of
   (a) providing a fusion protein of the target polypeptide and a labeling peptide and providing a sample comprising at least one ligand of interest;
   (b) incubating a liquid mixture comprising the fusion protein and the sample of step (a) under conditions allowing binding of the fusion protein to the sample;
   (c) incubating the liquid mixture of step (b) under suitable conditions to allow differential denaturation of the fusion protein bound to the sample and fusion protein not bound to the sample;
   (d) generating a signal from the fusion protein subjected to the denaturation of step (c) by adding to the mixture a second label suitable to react with the labeling peptide in the fusion protein;
   (e) detecting the signal in step (d) and the signal of a control comprising the steps (a) to (d) without providing the sample in step (a),
16. The method of any one of items 1 to 15, wherein step (b) comprises incubating at a temperature of 20 to 60°C for 1 to 70 min, preferably a temperature of 54°C for 30 min to 60 min.
17. The method of any one of items 1 to 16, wherein the denaturation in step (c) comprises heating the liquid mixture and at least one cooling step, wherein the heating is conducted at about 40°C to about 70°C.
18. The method of any one of items 1 to 17, wherein the denaturation is a step of heating the liquid mixture to a temperature of about 45°C to about 65°C, preferably of about 50°C to 60°C for a time period corresponding to 0.5 to 20 apparent half-life times of the antigen in the absence of ligand at the respective temperature.
19. The method of any one of items 1 to 18, wherein step (c) comprises a single isothermal heating step of the sample at a different temperature compared to a control.
20. The method of any one of items 1 to 21, wherein step (c) comprises a single non-isothermal heating step or several isothermal or non-isothermal heating steps interrupted by cooling steps.
21. The method of any one of items 1 to 20, wherein the sample is diluted with a ratio preferably of about 1:20, 1:50, 1:100.
22. The method on any one of items 1 to 21, wherein the sample is applied in a limiting dilution enabling determination of the limit of detection, preferably of about 1:10 to 1:1 000 000.
23. The method of any one of items 1 to 22, wherein the labeling peptide is an enzyme, a fluorescent peptide, a DNA probe or other biological marker, wherein the enzyme is preferably a luciferase or phosphatase.
24. The method of any one of items 1 to 23, wherein the method comprises additionally before step (a) and/or after step (e) conducting a further method for determining the immune status of a subject.
25. The method of any one of items 1 to 24, wherein the further method for determining the immune status of a subject comprises one or more solid-phase immune assay such as Western Blot or ELISA.
26. Use of the method of any one of items 1 to 25 for determining one or more of the following antibody maturation, vaccination status, antibody determination in acute, chronic or anamnestic infection, antibody effectiveness, and/or antibody avidity.
27. Use of the method of any one of items 1 to 25 for screening of antibodies, antibody fragments and/or immunoreceptors specific for a target polypeptide.
28. A kit comprising a mixture comprising a fusion protein of a target peptide and a labeling peptide for use in determining the binding property of an antibody for the target peptide.
29. The kit of item 28 for conducting the method of any one of items 1 to 25.

### REFERENCES

1. Brandts, J.F.; Lin, L.N. Study of Strong to Ultratight Protein Interactions Using Differential Scanning Calorimetry. Biochemistry 1990, 29, 6927-6940, doi:10.1021/bi00481a024.
2. Bischof, J.C.; He, X. Thermal Stability of Proteins. 2005, 33, 12-33, doi:10.1196/annals.1363.003.
3. Du, X.; Li, Y.; Xia, Y.L.; Ai, S.M.; Liang, J.; Sang, P.; Ji, X.L.; Liu, S.Q. Insights into protein-ligand interactions: Mechanisms, models, and methods. Int. J. Mol. Sci. 2016, 17, doi:10.3390/ijms17020144.
4. Hawe, A.; Sutter, M.; Jiskoot, W. Extrinsic fluorescent dyes as tools for protein characterization. Pharm. Res. 2008, 25, 1487-1499, doi:10.1007/s11095-007-9516-9.
5. Eftink, M.R. The use of fluorescence methods to monitor unfolding transitions in proteins. Biophys. J. 1994, 66, 482-501, doi:10.1016/s0006-3495(94)80799-4*.*
6. Pantoliano, M.W.; Petrella, E.C.; Kwasnoski, J.D.; Lobanov, V.S.; Myslik, J.; Graf, E.; Carver, T.; Asel, E.; Springer, B.A.; Lane, P.; et al. High-Density Miniaturized Thermal Shift Assays as a General Strategy for Drug Discovery. J. Biomol. Screen. 2001, 6, 429-440, doi:10,1177/108705710100600609.
7. Lo, M.C.; Aulabaugh, A.; Jin, G.; Cowling, R.; Bard, J.; Malamas, M.; Ellestad, G. Evaluation of fluorescence-based thermal shift assays for hit identification in drug discovery. Anal. Biochem. 2004, 332, 153-159, doi:10.1016/j.ab.2004.04.031.
8. Niesen, F.H.; Berglund, H.; Vedadi, M. The use of differential scanning fluorimetry to detect ligand interactions that promote protein stability. Nat. Protoc. 2007, 2, 2212-2221, doi:10.1038/nprot.2007.321.
9. Layton, C.J.; Hellinga, H.W. Thermodynamic analysis of ligand-induced changes in protein thermal unfolding applied to high-throughput determination of ligand affinities with extrinsic fluorescent dyes. Biochemistry 2010, 49, 10831-10841, doi:10.1021/bi101414z.
10. Layton, C.J.; Hellinga, H.W. Quantitation of protein-protein interactions by thermal stability shift analysis. Protein Sci. 2011, 20, 1439-1450, doi:10.1002/pro.674.
11. Ericsson, U.B.; Hallberg, B.M.; DeTitta, G.T.; Dekker, N.; Nordlund, P. Thermofluor-based high-throughput stability optimization of proteins for structural studies. Anal. Biochem. 2006, 357, 289-298, doi:10.1016/j.ab.2006.07.027.
12. Jafari, R.; Almqvist, H.; Axelsson, H.; Ignatushchenko, M.; Lundbäck, T.; Nordlund, P.; Molina, D.M. The cellular thermal shift assay for evaluating drug target interactions in cells. Nat. Protoc. 2014, 9, 2100-2122, doi:10.1038/nprot.2014.138.
13. Molina, D.M.; Jafari, R.; Ignatushchenko, M.; Seki, T.; Larsson, E.A.; Dan, C.; Sreekumar, L.; Cao, Y.; Nordlund, P. Monitoring drug target engagement in cells and tissues using the cellular thermal shift assay. Science. 2013, 341, 84-87, doi:10.1126/science.1233606.
14. Savitski, M.M.; Reinhard, F.B.M.; Franken, H.; Werner, T.; Savitski, M.F.; Eberhard, D.; Molina, D.M.; Jafari, R.; Dovega, R.B.; Klaeger, S.; et al. Tracking cancer drugs in living cells by thermal profiling of the proteome. Science. 2014, 346, doi:10.1126/science.1255784.
15. Tolvanen, T.A. Current Advances in CETSA. Front. Mol. Biosci. 2022, 9, doi:10.3389/fmolb.2022.866764.
16. Franken, H.; Mathieson, T.; Childs, D.; Sweetman, G.M.A.; Werner, T.; Tögel, I.; Doce, C.; Gade, S.; Bantscheff, M.; Drewes, G.; et al. Thermal proteome profiling for unbiased identification of direct and indirect drug targets using multiplexed quantitative mass spectrometry. Nat. Protoc. 2015, 10, 1567-1593, doi:10.1038/nprot.2015.101.
17. Jarzab, A.; Kurzawa, N.; Hopf, T.; Moerch, M.; Zecha, J.; Leijten, N.; Bian, Y.; Musiol, E.; Maschberger, M.; Stoehr, G.; et al. Meltome atlas-thermal proteome stability across the tree of life. Nat. Methods 2020, 17, 495-503, doi:10.1038/s41592-020-0801-4.
18. Henderson, M.J.; Holbert, M.A.; Simeonov, A.; Kallal, L.A. High-Throughput Cellular Thermal Shift Assays in Research and Drug Discovery. SLAS Discov. 2020, 25, 137-147, doi:10.1177/2472555219877183.
19. Massey, A.J. A high content, high throughput cellular thermal stability assay for measuring drug-target engagement in living cells. PLoS One 2018, 13, 1-17, doi:10.1371/journal.pone.0195050.
20. Almqvist, H.; Axelsson, H.; Jafari, R.; Dan, C.; Mateus, A.; Haraldsson, M.; Larsson, A.; Molina, D.M.; Artursson, P.; Lundbäck, T.; et al. CETSA screening identifies known and novel thymidylate synthase inhibitors and slow intracellular activation of 5-fluorouracil. Nat. Commun. 2016, 7, 1-11, doi:10.1038/ncomms11040.
21. Shaw, J.; Dale, I.; Hemsley, P.; Leach, L.; Dekki, N.; Orme, J.P.; Talbot, V.; Narvaez, A.J.; Bista, M.; Martinez Molina, D.; et al. Positioning High-Throughput CETSA in Early Drug Discovery through Screening against B-Raf and PARP1. SLAS Discov. 2019, 24, 121-132, doi:10.1177/2472555218813332.
22. Dart, M.L.; Machleidt, T.; Jost, E.; Schwinn, M.K.; Robers, M.B.; Shi, C.; Kirkland, T.A.; Killoran, M.P.; Wilkinson, J.M.; Hartnett, J.R.; et al. Homogeneous Assay for Target Engagement Utilizing Bioluminescent Thermal Shift. ACS Med. Chem. Lett. 2018, 9, 546-551, doi:10.1021/acsmedchemlett.8b00081.
23. Martinez, N.J.; Asawa, R.R.; Cyr, M.G.; Zakharov, A.; Urban, D.J.; Roth, J.S.; Wallgren, E.; Klumpp-Thomas, C.; Coussens, N.P.; Rai, G.; et al. A widely-applicable high-throughput cellular thermal shift assay (CETSA) using split Nano Luciferase. Sci. Rep. 2018, 8, doi: 1 0.1 038/s41598-018-27834-y.
24. England, C.G.; Ehlerding, E.B.; Cai, W. NanoLuc: A Small Luciferase Is Brightening Up the Field of Bioluminescence. Bioconjug. Chem. 2016, 27, 1175-1187, doi: 10.1021/acs.bioconjchem.6b00112.
25. McNulty, D.E.; Bonnette, W.G.; Qi, H.; Wang, L.; Ho, T.F.; Waszkiewicz, A.; Kallal, L.A.; Nagarajan, R.P.; Stern, M.; Quinn, A.M.; et al. A High-Throughput Dose-Response Cellular Thermal Shift Assay for Rapid Screening of Drug Target Engagement in Living Cells, Exemplified Using SMYD3 and IDO1. SLAS Discov. 2018, 23, 34-46, doi: 10.1177/2472555217732014.
26. Mortison, J.D.; Cornella-Taracido, I.; Venkatchalam, G.; Partridge, A.W.; Siriwardana, N.; Bushell, S.M. Rapid Evaluation of Small Molecule Cellular Target Engagement with a Luminescent Thermal Shift Assay. ACS Med. Chem. Lett. 2021, 12, 1288-1294, doi:10.1021/acsmedchemlett.1c00276.
27. Kirley, T.L.; Norman, A.B.; Wetzel, H.N. A novel differential scanning fluorimetry analysis of a humanized anti-cocaine mAb and its ligand binding characteristics. J. Immunol. Methods 2020, 476, doi:10.1016/j.jim.2019.112676.
28. Hedman, K.; Lappalainen, M.; Söderlund, M.; Hedman, L. Avidity of IgG in serodiagnosis of infectious diseases. Rev. Med. Microbiol. 1993, 4, 123-129, doi:10.1097/00013542-199307000-00001.
29. Friguet, B.; Chaffotte, A.F.; Djavadi-ohaniance, L.; Goldberg, M.E. Measurements of the true affinity constant in solution of antigen-antibody complexes by ELISA. J. Immunol. Methods 1985, 77, 305-319, doi:10.1016/0022-1759(85)90044-4.
30. Hedman, K.; Rousseau, S.A. Measurement of avidity of specific IgG for verification of recent primary rubella. J. Med. Virol. 1989, 27, 288-292, doi:10.1002/jmv.1890270406.
31. Thomas, H.I.J.; Morgan-Capner, P. Rubella-specific IgG subclass avidity ELISA and its role in the differentiation between primary rubella and rubella reinfection. Epidemiol. Infect. 1988, 101, 591-598, doi:10.1017/S0950268800029459.
32. Eisen, H.N.; Siskind, G.W. Variations in Affinities of Antibodies during the Immune Response. Biochemistry 1964, 3, 996-1008, doi:10.1021/bi00895a027.
33. Lynch, H.E.; Stewart, S.M.; Kepler, T.B.; Sempowski, G.D.; Alam, S.M. Surface plasmon resonance measurements of plasma antibody avidity during primary and secondary responses to anthrax protective antigen. J. Immunol. Methods 2014, 404, 1-12, doi:10.1016/j.jim.2013.11.026.
34. Classen, N.; Ulrich, D.; Hofemeier, A.; Hennies, M.T.; Hafezi, W.; Pettke, A.; Romberg, M.-L.; Lorentzen, E.U.; Hensel, A.; Kühn, J.E. Broadly Applicable, Virus-Free Dual Reporter Assay to Identify Compounds Interfering with Membrane Fusion: Performance for HSV-1 and SARS-CoV-2. Viruses 2022, 1354, doi:10.3390/v14071354.
35. Schoefbaenker, M.; Neddermeyer, R.; Guenther, T.; Mueller, M. M.; Romberg, M.-L.; Classen, N.; Hennies, M. T.; Hrincius, E. R.; Ludwig, S.; Kuehn, J. E.; Lorentzen, E. U. Surrogate Virus Neutralisation Test Based on Nanoluciferase-Tagged Antigens to Quantify Inhibitory Antibodies against SARS-CoV-2 and Characterise Omicron-Specific Reactivity in a Vaccination Cohort. Vaccines 2023, 11(12), 1832, doi:10.3390/vaccines11121832.
36. Hoffmann, M.; Kleine-Weber, H.; Schroeder, S.; Krüger, N.; Herrler, T.; Erichsen, S.; Schiergens, T.S.; Herrler, G.; Wu, N.H.; Nitsche, A.; et al. SARS-CoV-2 Cell Entry Depends on ACE2 and TMPRSS2 and Is Blocked by a Clinically Proven Protease Inhibitor. Cell 2020, 181, 271-280.e8, doi:10.1016/j.cell.2020.02.052.
37. Carreño, J.M.; Alshammary, H.; Tcheou, J.; Singh, G.; Raskin, A.J.; Kawabata, H.; Sominsky, L.A.; Clark, J.J.; Adelsberg, D.C.; Bielak, D.A.; et al. Activity of convalescent and vaccine serum against SARS-CoV-2 Omicron. Nature 2022, 602, 682-688, doi:10.1038/s41586-022-04399-5
38. Hoffmann, M.; Krüger, N.; Schulz, S.; Cossmann, A.; Rocha, C.; Kempf, A.; Nehlmeier, I.; Graichen, L.; Moldenhauer, A.S.; Winkler, M.S.; et al. The Omicron variant is highly resistant against antibody-mediated neutralization: Implications for control of the COVID-19 pandemic. Cell 2022, 185, 447-456.e11, doi:10.1016/j.cell.2021.12.032.
39. Bauer, G.; Struck, F.; Schreiner, P.; Staschik, E.; Soutschek, E.; & Motz, M. The challenge of avidity determination in SARS-CoV-2 serology. Journal of Medical Virology 2021, 93(5), 3092-3104, doi:10.1002/jmv.26863

## Claims

1. A method for determining the immune status of a subject, whether the subject has been in contact with a target polypeptide reactive with an antibody, which target polypeptide induces the production of said antibody in the subject, the method comprising subjecting a fusion protein between said target polypeptide and a labeling peptide (i) with and, as a control, (ii) without a sample obtained from said subject to a thermal shift assay; and detecting a signal from (i) and (ii),
wherein the ratio of the detected signal (i) and (ii) allows determining the binding characteristic between said antibody and said target polypeptide, and thereby the immune status of the subject is determined.

2. The method of claim 1, wherein a value of the ratio above a threshold is indicative of the production of the antibody as a result of a contact with the target polypeptide, preferably wherein the value of the ratio is in the range from 1 to 10000, preferably from 1.5 to 1000, further preferred from 1.5 to 500, in particular preferred also preferred from 1.5 to 100.

3. The method of any one of claims 1 or 2, wherein
(i) a value of the ratio in the range of less than 2 is indicative of antibodies which bind at a low level;
(ii) a value of the ratio in the range of equal or superior to 2 to equal or less than 30 is indicative of antibodies which bind at a mid-range level; and
(iii) a value of the ratio in the range of equal or superior to 30 is indicative of antibodies which bind at a high level.

4. The method of any one of claims 1 to 3, wherein
(i) is indicative of the immune status of a subject with a first contact with the target polypeptide at a first point in time t1,
(ii) is indicative of the immune status of a subject with a second contact with the target polypeptide at a second, third, or fourth point in time t2, t3, and/or t4, and
(iii) is indicative of the immune status of a subject with a third contact with the target polypeptide at a fifth point in time t5.

5. The method of any one of claims 1 to 4, wherein the antibody is a full-length antibody or an antibody fragment of human or animal origin, and is selected from polyclonal antibodies, monoclonal antibodies, nanobodies and antibody fragments such as Fab- or F(ab)₂-fragment.

6. The method of any one of claims 1 to 5, wherein determining the immune status comprises determining one or more characteristic of the antibody or antibody fragment selected from avidity, neutralizing capacity, concentration, titre, affinity, maturation, stability, preferably thermal stability, epitope mapping, and paratope mapping.

7. The method of any one of claims 1 to 6, wherein the target polypeptide is a monomer or a multimer of the target polypeptide, wherein the multimer comprises monomeric units of one or more variant-specific peptide sequences, preferably wherein the target polypeptide is selected from the group of viral polypeptide, bacterial polypeptide, fungal polypeptide, parasite polypeptide, allergenic polypeptides, synthetic polypeptides endogenous antigenic polypeptides such as peptide hormones, cytokines and polypeptides associated with tumors, degenerative or autoimmune disease, preferably wherein the target polypeptide is derived from a viral, bacterial, fungal or parasite polypeptides associated with an acute, chronic, persistent or anamnestic viral, bacterial, fungal or parasite infection in a subject, such as coronavirus infection, cytomegalovirus infection, parvovirus B19 infection, influenza virus infection, hepatitis virus infection, borreliosis or toxoplasmosis.

8. The method of any one of claims 1 to 7, preferably wherein the viral polypeptide derives from the group selected from dsDNA viruses, ssDNA viruses, dsRNA viruses, (+)ssRNA viruses, (-)ssRNA viruses, ssRNA-RT viruses, and dsDNA-RT viruses, preferably wherein the viral polypeptide is a (+)ssRNA virus-derived polypeptide, preferably a coronavirus polypeptide, preferably a SARS-CoV-2 polypeptide, preferably selected from SARS-CoV-2 spike protein, SARS-CoV-2 envelope protein, SARS-CoV-2 membrane protein, and SARS-CoV-2 nucleocapsid protein, preferably wherein the viral polypeptide is the RBD domain of the SARS-CoV-2 spike protein, preferably a single or double-RBD domain.

9. The method of any one of claims 1 to 8, wherein the thermal shift assay comprises the steps of
(a) providing a fusion protein of the target polypeptide and a labeling peptide and providing a sample comprising at least one ligand of interest;
(b) incubating a liquid mixture comprising the fusion protein and the sample of step (a) under conditions allowing binding of the fusion protein to the sample;
(c) incubating the liquid mixture of step (b) under suitable conditions to allow differential denaturation of the fusion protein bound to the sample and fusion protein not bound to the sample;
(d) generating a signal from the fusion protein subjected to the denaturation of step (c) by adding to the mixture a second label suitable to react with the labeling peptide in the fusion protein;
(e) detecting the signal in step (d) and the signal of a control comprising the steps (a) to (d) without providing the sample in step (a).

10. The method of any one of claims 1 to 9, wherein step (b) comprises incubating at a temperature of 20 to 60°C for 1 to 70 min, preferably a temperature of 54°C for 30 min to 60 min, preferably wherein the denaturation in step (c) comprises heating the liquid mixture and at least one cooling step, wherein the heating is conducted at about 40°C to about 70°C, preferably wherein the denaturation is a step of heating the liquid mixture to a temperature of about 45°C to about 65°C, preferably of about 50°C to 60°C for a time period corresponding to 0.5 to 20 apparent half-life times of the antigen in the absence of ligand at the respective temperature, preferably wherein step (c) comprises a single isothermal heating step of the sample at a different temperature compared to a control, preferably wherein step (c) comprises a single non-isothermal heating step or several isothermal or non-isothermal heating steps interrupted by cooling steps.

11. The method of any one of claims 1 to 10, wherein the sample is diluted with a ratio preferably of about 1:20, 1:50, 1:100, preferably wherein the sample is applied in a limiting dilution enabling determination of the limit of detection, preferably of about 1:10 to 1:1 000 000.

12. The method of any one of claims 1 to 11, wherein the labeling peptide is an enzyme, a fluorescent peptide, a DNA probe or other biological marker, wherein the enzyme is preferably a luciferase or phosphatase.

13. The method of any one of claims 1 to 12, wherein the method comprises additionally before step (a) and/or after step (e) conducting a further method for determining the immune status of a subject, preferably wherein the further method for determining the immune status of a subject comprises one or more solid-phase immune assay such as Western Blot or ELISA.

14. Use of the method of any one of claims 1 to 13 for determining one or more of the following antibody maturation, vaccination status, antibody determination in acute, chronic or anamnestic infection, antibody effectiveness, antibody affinity, and/or antibody avidity, preferably wherein the use is for screening of antibodies, antibody fragments and/or immunoreceptors specific for a target polypeptide.

15. A kit comprising a mixture comprising a fusion protein of a target peptide and a labeling peptide for use in determining the binding property of an antibody for the target peptide, preferably wherein the kit is for conducting the method of any one of claims 1 to 13.
